# EUROPEAN PATENT APPLICATION

(11) **EP 1 947 198 A1**
(43) Date of publication of application: **23.07.2008**
(21) Application number: 07290066.5
(22) Date of filing: 18.01.2007
(51) Int. Cl.: C12Q 1/68, C12N 15/82, A01H 5/00

(54) **Maize plants characterised by quantitative trait loci (QTL)**

(71) Applicant: Syngenta Participations AG, 4058 Basel (CH)
(72) Inventor: Ragot, Michel, 31000 Toulouse (FR); Lespinasse, Denis, 31340 La Magdelaine sur Tarn (FR); Muller, Jean-Paul, 50220 Ducey (FR); Delage, Pascal, 35610 Roz-Sur-Couesnon (FR)
(74) Representative: Vossius & Partner

(57) **Abstract**

The present invention relates to maize plants with a genome comprising a unique allele profile associated with the corresponding QTLs contributing to the expression of a variety of phenotypic traits of economic interest selected from the group of grain yield, grain moisture at harvest, early and late root lodging, stalk lodging, common smut incidence, fusarium ear rot incidence, sulcotrione resistance, and tassel architecture.

The invention further relates to method for obtaining such a plant as well as assays and screening methods for identifying plants with the desired profile.

### Annex to the application documents - subsequently filed sequences listing

SEQUENCE LISTING

<110>  Syngenta Participations AG

<120>  Novel Maize Plant

<130>  M3047 EP BS

<140>  07 29 0066.5
<141>  2007-01-18

<160>  82

<170>  PatentIn version 3.4

<210>  1
<211>  18
<212>  DNA
<213>  Artificial sequence

<220>
<223>  forward primer 1

<400>  1
agaaaatggt gaggcagg                                                    18


<210>  2
<211>  24
<212>  DNA
<213>  Artificial sequence

<220>
<223>  reverse primer 1

<400>  2
tatgaaatct gcatctagaa attg                                             24


<210>  3
<211>  22
<212>  DNA
<213>  Artificial sequence

<220>
<223>  forward primer 2

<400>  3
tgacggacgt ggatcgcttc ac                                               22


<210>  4
<211>  22
<212>  DNA
<213>  Artificial sequence

<220>
<223>  reverse primer 2

<400>  4
agcaggcagc aggtcagcag cg                                               22


<210>  5
<211>  24
<212>  DNA
<213>  Artificial sequence

<220>
<223>  forward primer 3

<400>  5
taattcctcg ctcccggatt cagc                                              24


<210>  6
<211>  25
<212>  DNA
<213>  Artificial sequence

<220>
<223>  reverse primer 3

<400>  6
gtgcatgagg gagcagcagg tagtg                                             25


<210>  7
<211>  22
<212>  DNA
<213>  Artificial sequence

<220>
<223>  forward primer 4

<400>  7
gacgtaagcc tagctctgcc at                                                22


<210>  8
<211>  24
<212>  DNA
<213>  Artificial sequence

<220>
<223>  reverse primer 4

<400>  8
aaacaagaac ggcggtgctg attc                                              24


<210>  9
<211>  26
<212>  DNA
<213>  Artificial sequence

<220>
<223>  forward primer 5

<400>  9
ccgaattgaa atagctgcga gaacct                                            26


<210>  10
<211>  26
<212>  DNA
<213>  Artificial sequence

<220>
<223>  reverse primer 5

<400>  10
acaatgaacg gtggttatca acacgc                                            26


<210>  11

<211>  26
<212>  DNA
<213>  Artificial sequence

<220>
<223>  forward primer 6

<400>  11
ttactcctat ccactgcggc ctggac                                          26


<210>  12
<211>  23
<212>  DNA
<213>  Artificial sequence

<220>
<223>  reverse primer 6

<400>  12
gcggcatccc gtacagcttc aga                                             23


<210>  13
<211>  27
<212>  DNA
<213>  Artificial sequence

<220>
<223>  forward primer 7

<400>  13
ctgctctcac tgagcttgat ggaaagg                                         27


<210>  14
<211>  30
<212>  DNA
<213>  Artificial sequence

<220>
<223>  reverse primer 7

<400>  14
tgcaaatcaa tggcaaggga cctcgtagtt                                      30


<210>  15
<211>  21
<212>  DNA
<213>  Artificial sequence

<220>
<223>  forward primer 8

<400>  15
catgcatcaa cgtaactccc t                                               21


<210>  16
<211>  20
<212>  DNA
<213>  Artificial sequence

<220>
<223>  reverse primer 8

<400>  16

catgtcacgc gttccacttg                                                20

<210>  17
<211>  18
<212>  DNA
<213>  Artificial sequence

<220>
<223>  forward primer 9

<400>  17
cttccagccg caaccctc                                                  18

<210>  18
<211>  18
<212>  DNA
<213>  Artificial sequence

<220>
<223>  reverse primer 9

<400>  18
ccaacaacgc ggacgtga                                                  18

<210>  19
<211>  19
<212>  DNA
<213>  Artificial sequence

<220>
<223>  forward primer 10

<400>  19
ggtcaccctc cctttgcag                                                 19

<210>  20
<211>  22
<212>  DNA
<213>  Artificial sequence

<220>
<223>  reverse primer 10

<400>  20
attgcctaca cagtttgatt gg                                             22

<210>  21
<211>  25
<212>  DNA
<213>  Artificial sequence

<220>
<223>  forward primer 11

<400>  21
gcaaaccttg catgaacccg attgt                                          25

<210>  22
<211>  23
<212>  DNA
<213>  Artificial sequence

<220>
<223>  reverse primer 11

<400>  22
caagcgtcca gctcgatgat ttc                                              23


<210>  23
<211>  20
<212>  DNA
<213>  Artificial sequence

<220>
<223>  forward primer 12

<400>  23
gatgcaataa aggttgccgt                                                  20


<210>  24
<211>  18
<212>  DNA
<213>  Artificial sequence

<220>
<223>  reverse primer 12

<400>  24
atgtgctgtg cctgcctc                                                    18


<210>  25
<211>  20
<212>  DNA
<213>  Artificial sequence

<220>
<223>  forward primer 13

<400>  25
gggagtatgg tagggaaccc                                                  20


<210>  26
<211>  20
<212>  DNA
<213>  Artificial sequence

<220>
<223>  reverse primer 13

<400>  26
aaacccttgg agcataccct                                                  20


<210>  27
<211>  18
<212>  DNA
<213>  Artificial sequence

<220>
<223>  forward primer 14

<400>  27
ttcaccgcct cacatgac                                                    18


<210>  28

<211>  19
<212>  DNA
<213>  Artificial sequence

<220>
<223>  reverse primer 14

<400>  28
gcaacgctag ctagctttg                                                    19


<210>  29
<211>  20
<212>  DNA
<213>  Artificial sequence

<220>
<223>  forward primer 15

<400>  29
ttccagtaag ggaggtgctg                                                   20


<210>  30
<211>  20
<212>  DNA
<213>  Artificial sequence

<220>
<223>  reverse primer 15

<400>  30
taagcaacat atagccgggc                                                   20


<210>  31
<211>  20
<212>  DNA
<213>  Artificial sequence

<220>
<223>  forward primer 16

<400>  31
gtgaagaacg atgacgcaga                                                   20


<210>  32
<211>  20
<212>  DNA
<213>  Artificial sequence

<220>
<223>  reverse primer 16

<400>  32
cagcaacgct ctcacattgt                                                   20


<210>  33
<211>  23
<212>  DNA
<213>  Artificial sequence

<220>
<223>  forward primer 17

<400>  33

cagagttgat gaactgaaaa agg                                                23

<210>  34
<211>  20
<212>  DNA
<213>  Artificial sequence

<220>
<223>  reverse primer 17

<400>  34
ctcttgcttc ccccctaatc                                                     20

<210>  35
<211>  20
<212>  DNA
<213>  Artificial sequence

<220>
<223>  forward primer 18

<400>  35
cgttacccat tcctgctacg                                                     20

<210>  36
<211>  20
<212>  DNA
<213>  Artificial sequence

<220>
<223>  reverse primer 18

<400>  36
cttgctcgtt tccattccat                                                     20

<210>  37
<211>  20
<212>  DNA
<213>  Artificial sequence

<220>
<223>  forward primer 19

<400>  37
agctgatctg cacgttgttg                                                     20

<210>  38
<211>  20
<212>  DNA
<213>  Artificial sequence

<220>
<223>  reverse primer 19

<400>  38
gcagatccac gccatttaaa                                                     20

<210>  39
<211>  20
<212>  DNA
<213>  Artificial sequence

<220>
<223>  forward primer 20

<400>  39
acaattcgat cgagagcgag                                                    20


<210>  40
<211>  20
<212>  DNA
<213>  Artificial sequence

<220>
<223>  reverse primer 20

<400>  40
cctttcttgc tggttcttgc                                                    20


<210>  41
<211>  25
<212>  DNA
<213>  Artificial sequence

<220>
<223>  forward primer 21

<400>  41
ttttctttca aaaatattca gaagc                                              25


<210>  42
<211>  20
<212>  DNA
<213>  Artificial sequence

<220>
<223>  reverse primer 21

<400>  42
gcaggatttc atcggttgtt                                                    20


<210>  43
<211>  20
<212>  DNA
<213>  Artificial sequence

<220>
<223>  forward primer 22

<400>  43
ctgggcagac agcaacagta                                                    20


<210>  44
<211>  20
<212>  DNA
<213>  Artificial sequence

<220>
<223>  reverse primer 22

<400>  44
agccaaagac atgatggtcc                                                    20


<210>  45

<211>  20
<212>  DNA
<213>  Artificial sequence

<220>
<223>  forward primer 23

<400>  45
aggtcctggc actaagagca                                                    20


<210>  46
<211>  21
<212>  DNA
<213>  Artificial sequence

<220>
<223>  reverse primer 23

<400>  46
agaggtggta tgatcacctg g                                                  21


<210>  47
<211>  20
<212>  DNA
<213>  Artificial sequence

<220>
<223>  forward primer 24

<400>  47
cagaagggga ggagggatac                                                    20


<210>  48
<211>  20
<212>  DNA
<213>  Artificial sequence

<220>
<223>  reverse primer 24

<400>  48
attatgctca agcacagggc                                                    20


<210>  49
<211>  20
<212>  DNA
<213>  Artificial sequence

<220>
<223>  forward primer 25

<400>  49
aaccaaggtt cttggaggct                                                    20


<210>  50
<211>  25
<212>  DNA
<213>  Artificial sequence

<220>
<223>  reverse primer 25

<400>  50

accattgtat tttcctagag aatcg                                          25

<210>  51
<211>  20
<212>  DNA
<213>  Artificial sequence

<220>
<223>  forward primer 26

<400>  51
taatcttggg gggtttaggg                                               20

<210>  52
<211>  20
<212>  DNA
<213>  Artificial sequence

<220>
<223>  reverse primer 26

<400>  52
gacatgtccc attcccattc                                               20

<210>  53
<211>  20
<212>  DNA
<213>  Artificial sequence

<220>
<223>  forward primer 27

<400>  53
acgactttca tgcctcgtct                                               20

<210>  54
<211>  20
<212>  DNA
<213>  Artificial sequence

<220>
<223>  reverse primer 27

<400>  54
atttcttttg ccacctcagc                                               20

<210>  55
<211>  21
<212>  DNA
<213>  Artificial sequence

<220>
<223>  forward primer 28

<400>  55
ttttctcctt gagttcgttc g                                             21

<210>  56
<211>  20
<212>  DNA
<213>  Artificial sequence

<220>
<223>  reverse primer 28

<400>  56
acaggcagag ctctcacaca                                                   20


<210>  57
<211>  24
<212>  DNA
<213>  Artificial sequence

<220>
<223>  forward primer 29

<400>  57
acagctttag acttagacca cacg                                              24


<210>  58
<211>  20
<212>  DNA
<213>  Artificial sequence

<220>
<223>  reverse primer 29

<400>  58
gcacaagcga aggttttctc                                                   20


<210>  59
<211>  19
<212>  DNA
<213>  Artificial sequence

<220>
<223>  forward primer 30

<400>  59
cccagcgcat gtcaactct                                                    19


<210>  60
<211>  20
<212>  DNA
<213>  Artificial sequence

<220>
<223>  reverse primer 30

<400>  60
ccccggtaat tcagtggata                                                   20


<210>  61
<211>  20
<212>  DNA
<213>  Artificial sequence

<220>
<223>  forward primer 31

<400>  61
tgctctcaca agatggtgga                                                   20


<210>  62

<211>  20
<212>  DNA
<213>  Artificial sequence

<220>
<223>  reverse primer 31

<400>  62
ccacaggata aaatcggctg                                                    20


<210>  63
<211>  20
<212>  DNA
<213>  Artificial sequence

<220>
<223>  forward primer 32

<400>  63
accggaacag acgagctcta                                                    20


<210>  64
<211>  20
<212>  DNA
<213>  Artificial sequence

<220>
<223>  reverse primer 32

<400>  64
gtcctgcaaa gcaacctagc                                                    20


<210>  65
<211>  20
<212>  DNA
<213>  Artificial sequence

<220>
<223>  forward primer 33

<400>  65
acagcactgg gaaccaaaac                                                    20


<210>  66
<211>  20
<212>  DNA
<213>  Artificial sequence

<220>
<223>  reverse primer 33

<400>  66
atcccctctt ccatctctgc                                                    20


<210>  67
<211>  20
<212>  DNA
<213>  Artificial sequence

<220>
<223>  forward primer 34

<400>  67

ttggtgaaac ggtgaaatga 20

<210> 68
<211> 20
<212> DNA
<213> Artificial sequence

<220>
<223> reverse primer 34

<400> 68
ctggtgagct tcaccctctc 20

<210> 69
<211> 20
<212> DNA
<213> Artificial sequence

<220>
<223> forward primer 35

<400> 69
ttacggtacc aattcgctcc 20

<210> 70
<211> 20
<212> DNA
<213> Artificial sequence

<220>
<223> reverse primer 35

<400> 70
gacgacgcca ttttctgatt 20

<210> 71
<211> 21
<212> DNA
<213> Artificial sequence

<220>
<223> forward primer 36

<400> 71
gagaagaggt ggacaaactc t 21

<210> 72
<211> 21
<212> DNA
<213> Artificial sequence

<220>
<223> reverse primer 36

<400> 72
tggaggtaga agagaattgt g 21

<210> 73
<211> 22
<212> DNA
<213> Artificial sequence

<220>
<223>  forward primer 37

<400>  73
tcgtcgtctc caatcatacg tg                                              22


<210>  74
<211>  22
<212>  DNA
<213>  Artificial sequence

<220>
<223>  reverse primer 37

<400>  74
gctacacgat accatggcgt tt                                              22


<210>  75
<211>  21
<212>  DNA
<213>  Artificial sequence

<220>
<223>  forward primer 38

<400>  75
atatcttctt cttgtcctcc g                                               21


<210>  76
<211>  17
<212>  DNA
<213>  Artificial sequence

<220>
<223>  reverse primer 38

<400>  76
catcccctta tccctcc                                                    17


<210>  77
<211>  24
<212>  DNA
<213>  Artificial sequence

<220>
<223>  forward primer 39

<400>  77
ttgcaccccg ttattatcct acag                                            24


<210>  78
<211>  24
<212>  DNA
<213>  Artificial sequence

<220>
<223>  reverse primer 39

<400>  78
ccagactaga gtgccatgat cctt                                            24


<210>  79

<211>  19
<212>  DNA
<213>  Artificial sequence

<220>
<223>  forward primer 40

<400>  79
agctcgagta cctgccgag                                                    19


<210>  80
<211>  15
<212>  DNA
<213>  Artificial sequence

<220>
<223>  reverse primer 40

<400>  80
tgcatctctg agacc                                                        15


<210>  81
<211>  21
<212>  DNA
<213>  Artificial sequence

<220>
<223>  forward primer 41

<400>  81
tggtcttctt cgccgcatta t                                                 21


<210>  82
<211>  24
<212>  DNA
<213>  Artificial sequence

<220>
<223>  reverse primer 41

<400>  82
ataagctcgt tgatctcctc ctcc                                              24

## Description

The subject matter of the present invention relates to plants, particularly to maize plants with a genome comprising a unique allele profile associated with the corresponding QTLs contributing to the expression of a variety of phenotypic traits of economic interest selected from the group of grain yield, grain moisture at harvest, early and late root lodging, stalk lodging, common smut incidence, fusarium ear rot incidence, sulcotrione resistance, and tassel architecture.

The invention further relates to method for obtaining such a plant as well as assays and screening methods for identifying plants with the desired profile.

Selective breeding has been employed for centuries to improve, or attempt to improve, phenotypic traits of agronomic and economic interest in plants such as yield, percentage of grain oil, etc. Generally speaking, selective breeding involves the selection of individuals to serve as parents of the next generation on the basis of one or more phenotypic traits of interest. However, such phenotypic selection is frequently complicated by non-genetic factors that can impact the phenotype(s) of interest. Non-genetic factors that can have such effects include, but are not limited to environmental influences such as soil type and quality, rainfall, temperature range, and others.

Most phenotypic traits of interest are controlled by more than one genetic locus, each of which typically influences the given trait to a greater or lesser degree. For example, U.S. Patent NO: 6,399,855 to Beavis suggests that the vast majority of economically important phenotypic traits in domesticated plants are so-called quantitative traits. Generally, the term "quantitative trait" has been used to describe a phenotype that exhibits continuous variability in expression and is the net result of multiple genetic loci presumably interacting with each other and/or with the environment. The term "complex trait" has also been broadly used to describe any trait that does not exhibit classic Mendelian inheritance, which generally is attributable to a single genetic locus (Lander & Schork (1994) 265 Science 2037-2048).

One of the consequences of multifactorial inheritance patterns is that it can be very difficult to map loci that contribute to the expression of such traits. However, the development of sets of polymorphic genetic markers (e.g., RFLPs, SNPs, SSRs, etc.) that span the genome has made it possible to investigate what Edwards et al. (1987) 115 Genetics 113-125 referred to as "quantitative trait loci" (QTL or QTLs), as well as their numbers, magnitudes, and distributions. QTLs include genes that control, to some degree, qualitative and quantitative phenotypic traits that can be discrete or continuously distributed within a family of individuals as well as within a population of families of individuals.

Various experimental approaches have been developed to identify and analyze QTLs *(see e.g.,* U.S. Patent Nos. 5,385,835; 5,492,547; and 5,981,832). One such approach involves crossing two inbred lines to produce F₁ single cross hybrid progeny, selfing the F₁ hybrid progeny to produce segregating F₂ progeny, genotyping multiple marker loci, and evaluating one to several quantitative phenotypic traits among the segregating progeny. The QTLs are then identified on the basis of significant statistical associations between the genotypic values and the phenotypic variability among the segregating progeny. This experimental paradigm is ideal in that the parental lines of the F₁ generation have known linkage phases, all of the segregating loci in the progeny are informative, and linkage disequilibrium between the marker loci and the genetic loci affecting the phenotypic traits is maximized.

In the present invention a commonly-used generation advancement procedure was applied to develop a maize plant which exhibits a unique allele profile at specific QTLs.

### Definitions

As used in this specification and the appended claims, the singular forms "a", "an", and "the" include plural referents unless the context clearly dictates otherwise. Thus, for example, reference to "a plant" includes one or more plants, and reference to "a cell" includes mixtures of cells, tissues, and the like.

An "allele" is understood within the scope of the invention to refer to alternative forms of various genetic units associated with different forms of a gene or of any kind of identifiable genetic element, which are alternative in inheritance because they are situated at the same locus in homologous chromosomes. In a diploid cell or organism, the two alleles of a given gene (or marker) typically occupy corresponding loci on a pair of homologous chromosomes.

An allele associated with a quantitative trait may comprise a single gene or multiple genes or even a gene encoding a genetic factor contributing to the phenotype represented by said QTL.

As used herein, the term "breeding", and grammatical variants thereof, refer to any process that generates a progeny individual. Breedings can be sexual or asexual, or any combination thereof. Exemplary non-limiting types of breedings include crossings, selfings, doubled haploid derivative generation, and combinations thereof.

As used herein, the phrase "established breeding population" refers to a collection of potential breeding partners produced by and/or used as parents in a breeding program; e.g., a commercial breeding program. The members of the established breeding population are typically well-characterized genetically and/or phenotypically. For example, several phenotypic traits of interest might have been evaluated, e.g., under different environmental conditions, at multiple locations, and/or at different times. Alternatively or in addition, one or more genetic loci associated with expression of the phenotypic traits might have been identified and one or more of the members of the breeding population might have been genotyped with respect to the one or more genetic loci as well as with respect to one or more genetic markers that are associated with the one or more genetic loci.

As used herein, the phrase "diploid individual" refers to an individual that has two sets of chromosomes, typically one from each of its two parents. However, it is understood that in some embodiments a diploid individual can receive its "maternal" and "paternal" sets of chromosomes from the same single organism, such as when a plant is selfed to produce a subsequent generation of plants.

"Homozygous" is understood within the scope of the invention to refer to like alleles at one or more corresponding loci on homologous chromosomes.

"Heterozygous" is understood within the scope of the invention to refer to unlike alleles at one or more corresponding loci on homologous chromosomes.

"Backcrossing" is understood within the scope of the invention to refer to a process in which a hybrid progeny is repeatedly crossed back to one of the parents.

"Locus" is understood within the scope of the invention to refer to a region on a chromosome, which comprises a gene contributing to a trait.

"Genetic linkage" is understood within the scope of the invention to refer to an association of characters in inheritance due to location of genes in proximity on the same chromosome, measured by percent recombination between loci (centi-Morgan, cM).

As used herein, the phrase "quantitative trait" refers to a phenotypic trait that can be described numerically (*i.e.,* quantitated or quantified). A quantitative trait typically exhibits continuous variation between individuals of a population; that is, differences in the numerical value of the phenotypic trait are slight and grade into each other. Frequently, the frequency distribution in a population of a quantitative phenotypic trait exhibits a bell-shaped curve (*i.e.,* exhibits a normal distribution between two extremes). A quantitative trait is typically the result of a genetic locus interacting with the environment or of multiple genetic loci (QTL) interacting with each other and/or with the environment. Examples of quantitative traits include plant height and yield.

As used herein, the terms "quantitative trait locus" (QTL) and "marker trait association" refer to an association between a genetic marker and a chromosomal region and/or gene that affects the phenotype of a trait of interest. Typically, this is determined statistically; e.g., based on one or more methods published in the literature. A QTL can be a chromosomal region and/or a genetic locus with at least two alleles that differentially affect the expression of a phenotypic trait (either a quantitative trait or a qualitative trait).

As used herein, the phrases "sexually crossed" and "sexual reproduction" in the context of the presently disclosed subject matter refers to the fusion of gametes to produce progeny (e.g., by fertilization, such as to produce seed by pollination in plants). A "sexual cross" or "cross-fertilization" is in some embodiments fertilization of one individual by another (e.g., cross-pollination in plants). The term "selfing" refers in some embodiments to the production of seed by self-fertilization or self-pollination; *i.e.,* pollen and ovule are from the same plant.

As used herein, the phrase "genetic marker" refers to a feature of an individual's genome (e.g., a nucleotide or a polynucleotide sequence that is present in an individual's genome) that is associated with one or more loci of interest. In some embodiments, a genetic marker is polymorphic in a population of interest, or the locus occupied by the polymorphism, depending on context. Genetic markers include, for example, single nucleotide polymorphisms (SNPs), indels (*i.e.,* insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples. Genetic markers can, for example, be used to locate genetic loci containing alleles that contribute to variability in expression of phenotypic traits on a chromosome. The phrase "genetic marker" can also refer to a polynucleotide sequence complementary to a genomic sequence, such as a sequence of a nucleic acid used as probes.

A genetic marker can be physically located in a position on a chromosome that is within or outside of to the genetic locus with which it is associated (*i.e.,* is intragenic or extragenic, respectively). Stated another way, whereas genetic markers are typically employed when the location on a chromosome of the gene that corresponds to the locus of interest has not been identified and there is a non-zero rate of recombination between the genetic marker and the locus of interest, the presently disclosed subject matter can also employ genetic markers that are physically within the boundaries of a genetic locus (e.g., inside a genomic sequence that corresponds to a gene such as, but not limited to a polymorphism within an intron or an exon of a gene). In some embodiments of the presently disclosed subject matter, the one or more genetic markers comprise between one and ten markers, and in some embodiments the one or more genetic markers comprise more than ten genetic markers.

As used herein, the term "genotype" refers to the genetic constitution of a cell or organism. An individual's "genotype for a set of genetic markers" includes the specific alleles, for one or more genetic marker loci, present in the individual. As is known in the art, a genotype can relate to a single locus or to multiple loci, whether the loci are related or unrelated and/or are linked or unlinked. In some embodiments, an individual's genotype relates to one or more genes that are related in that the one or more of the genes are involved in the expression of a phenotype of interest (e.g., a quantitative trait as defined herein). Thus, in some embodiments a genotype comprises a summary of one or more alleles present within an individual at one or more genetic loci of a quantitative trait. In some embodiments, a genotype is expressed in terms of a haplotype (defined herein below).

As used herein, the term "germplasm" refers to the totality of the genotypes of a population or other group of individuals (e.g., a species). The term "germplasm" can also refer to plant material; e.g., a group of plants that act as a repository for various alleles. The phrase "adapted germplasm" refers to plant materials of proven genetic superiority; e.g., for a given environment or geographical area, while the phrases "non-adapted germplasm," "raw germplasm," and "exotic germplasm" refer to plant materials of unknown or unproven genetic value; e.g., for a given environment or geographical area; as such, the phrase "non-adapted germplasm" refers in some embodiments to plant materials that are not part of an established breeding population and that do not have a known relationship to a member of the established breeding population.

As used herein, the term "haplotype" refers to the set of alleles an individual inherited from one parent. A diploid individual thus has two haplotypes. The term "haplotype" can be used in a more limited sense to refer to physically linked and/or unlinked genetic markers (e.g., sequence polymorphisms) associated with a phenotypic trait. The phrase "haplotype block" (sometimes also referred to in the literature simply as a haplotype) refers to a group of two or more genetic markers that are physically linked on a single chromosome (or a portion thereof). Typically, each block has a few common haplotypes, and a subset of the genetic markers (*i.e.*, a "haplotype tag") can be chosen that uniquely identifies each of these haplotypes.

As used herein, the terms "hybrid", "hybrid plant," and "hybrid progeny" refers to an individual produced from genetically different parents (e.g., a genetically heterozygous or mostly heterozygous individual).

If two individuals possess the same allele at a particular locus, the alleles are termed "identical by descent" if the alleles were inherited from one common ancestor (*i.e.,* the alleles are copies of the same parental allele). The alternative is that the alleles are "identical by state" (*i.e.,* the alleles appear the same but are derived from two different copies of the allele). Identity by descent information is useful for linkage studies; both identity by descent and identity by state information can be used in association studies such as those described herein, although identity by descent information can be particularly useful.

As used herein, the phrase "single cross F₁ hybrid" refers to an F₁ hybrid produced from a cross between two inbred lines.

As used herein, the phrase "inbred line" refers to a genetically homozygous or nearly homozygous population. An inbred line, for example, can be derived through several cycles of brother/sister breedings or of selfing. In some embodiments, inbred lines breed true for one or more phenotypic traits of interest. An "inbred", "inbred individual", or "inbred progeny" is an individual sampled from an inbred line.

As used herein, the term "linkage", and grammatical variants thereof, refers to the tendency of alleles at different loci on the same chromosome to segregate together more often than would be expected by chance if their transmission were independent, in some embodiments as a consequence of their physical proximity.

As used herein, the phrase "linkage disequilibrium" (also called "allelic association") refers to a phenomenon wherein particular alleles at two or more loci tend to remain together in linkage groups when segregating from parents to offspring with a greater frequency than expected from their individual frequencies in a given population. For example, a genetic marker allele and a QTL allele can show linkage disequilibrium when they occur together with frequencies greater than those predicted from the individual allele frequencies. Linkage disequilibrium can occur for several reasons including, but not limited to the alleles being in close proximity on a chromosome

As used herein, the term "locus" refers to a position on a chromosome (e.g., of a gene, a genetic marker, or the like).

As used herein, the phrase "nucleic acid" refers to any physical string of monomer units that can be corresponded to a string of nucleotides, including a polymer of nucleotides (e.g., a typical DNA or RNA polymer), modified oligonucleotides (e.g., oligonucleotides comprising bases that are not typical to biological RNA or DNA, such as 2'-*O-*methylated oligonucleotides), and the like. In some embodiments, a nucleic acid can be single-stranded, double-stranded, multi-stranded, or combinations thereof. Unless otherwise indicated, a particular nucleic acid sequence of the presently disclosed subject matter optionally comprises or encodes complementary sequences, in addition to any sequence explicitly indicated.

As used herein, the phrase "phenotypic trait" refers to the appearance or other detectable characteristic of an individual, resulting from the interaction of its genome with the environment.

As used herein, the term "plurality" refers to more than one. Thus, a "plurality of individuals" refers to at least two individuals. In some embodiments, the term plurality refers to more than half of the whole. For example, in some embodiments a "plurality of a population" refers to more than half the members of that population.

As used herein, the term "progeny" refers to the descendant(s) of a particular cross. Typically, progeny result from breeding of two individuals, although some species (particularly some plants and hermaphroditic animals) can be selfed (*i.e.,* the same plant acts as the donor of both male and female gametes). The descendant(s) can be, for example, of the F₁, the F₂, or any subsequent generation.

As used herein, the phrase "qualitative trait" refers to a phenotypic trait that is controlled by one or a few genes that exhibit major phenotypic effects. Because of this, qualitative traits are typically simply inherited. Examples in plants include, but are not limited to, flower color, cob color, and disease resistance such as Northern corn leaf blight resistance.

"Marker-based selection" is understood within the scope of the invention to refer to the use of genetic markers to detect one or more nucleic acids from the plant, where the nucleic acid is associated with a desired trait to identify plants that carry genes for desirable (or undesirable) traits, so that those plants can be used (or avoided) in a selective breeding program.

"Microsatellite or SSRs (Simple sequence repeats) (Marker)" is understood within the scope of the invention to refer to a type of genetic marker that consists of numerous repeats of short sequences of DNA bases, which are found at loci throughout the plant's DNA and have a likelihood of being highly polymorphic.

"PCR (Polymerase chain reaction)" is understood within the scope of the invention to refer to a method of producing relatively large amounts of specific regions of DNA, thereby making possible various analyses that are based on those regions.

"PCR primer" is understood within the scope of the invention to refer to relatively short fragments of single-stranded DNA used in the PCR amplification of specific regions of DNA.

"Phenotype" is understood within the scope of the invention to refer to a distinguishable characteristic(s) of a genetically controlled trait.

"Polymorphism" is understood within the scope of the invention to refer to the presence in a population of two or more different forms of a gene, genetic marker, or inherited trait.

"Selective breeding" is understood within the scope of the invention to refer to a program of breeding that uses plants that possess or display desirable traits as parents.

"Tester" plant" is understood within the scope of the invention to refer to a plant used to characterize genetically a trait in a plant to be tested. Typically, the plant to be tested is crossed with a "tester" plant and the segregation ratio of the trait in the progeny of the cross is scored.

As used herein, the term "tester" refers to a line or individual with a standard genotype, known characteristics, and established performance. A "tester parent" is an individual from a tester line that is used as a parent in a sexual cross. Typically, the tester parent is unrelated to and genetically different from the individual to which it is crossed. A tester is typically used to generate F₁ progeny when crossed to individuals or inbred lines for phenotypic evaluation.

As used herein, the phrase "topcross combination" refers to the process of crossing a single tester line to multiple lines. The purpose of producing such crosses is to determine phenotypic performance of hybrid progeny; that is, to evaluate the ability of each of the multiple lines to produce desirable phenotypes in hybrid progeny derived from the line by the tester cross.

The term "hybridize" as used herein refers to conventional hybridization conditions, preferably to hybridization conditions at which 5xSSPE, 1% SDS, 1xDenhardts solution is used as a solution and/or hybridization temperatures are between 35°C and 70°C, preferably 65°C. After hybridization, washing is preferably carried out first with 2xSSC, 1% SDS and subsequently with 0.2xSSC at temperatures between 35°C and 75°C, particularly between 45°C and 65°C, but especially at 59°C (regarding the definition of SSPE, SSC and Denhardts solution see Sambrook et al. loc. cit.). High stringency hybridization conditions as for instance described in Sambrook et al, supra, are particularly preferred. Particularly preferred stringent hybridization conditions are for instance present if hybridization and washing occur at 65°C as indicated above. Non-stringent hybridization conditions for instance with hybridization and washing carried out at 45°C are less preferred and at 35°C even less.

"Sequence Homology or Sequence identity" is used herein interchangeably. The terms "identical" or percent "identity" in the context of two or more nucleic acid or protein sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. If two sequences which are to be compared with each other differ in length, sequence identity preferably relates to the percentage of the nucleotide residues of the shorter sequence which are identical with the nucleotide residues of the longer sequence. Sequence identity can be determined conventionally with the use of computer programs such as the Bestfit program (Wisconsin Sequence Analysis Package, Version 8 for Unix, Genetics Computer Group, University Research Park, 575 Science Drive Madison, Wl 53711). Bestfit utilizes the local homology algorithm of Smith and Waterman, Advances in Applied Mathematics 2 (1981), 482-489, in order to find the segment having the highest sequence identity between two sequences. When using Bestfit or another sequence alignment program to determine whether a particular sequence has for instance 95% identity with a reference sequence of the present invention, the parameters are preferably so adjusted that the percentage of identity is calculated over the entire length of the reference sequence and that homology gaps of up to 5% of the total number of the nucleotides in the reference sequence are permitted. When using Bestfit, the so-called optional parameters are preferably left at their preset ("default") values. The deviations appearing in the comparison between a given sequence and the above-described sequences of the invention may be caused for instance by addition, deletion, substitution, insertion or recombination. Such a sequence comparison can preferably also be carried out with the program "fasta20u66" (version 2.0u66, September 1998 by William R. Pearson and the University of Virginia; see also W.R. Pearson (1990), Methods in Enzymology 183, 63-98, appended examples and http://workbench.sdsc.edu/). For this purpose, the "default" parameter settings may be used.

Another indication that two nucleic acid sequences are substantially identical is that the two molecules hybridize to each other under stringent conditions. The phrase: "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridizations are sequence dependent, and are different under different environmental parameters. Longer sequences hybridize specifically at higher temperatures. An extensive guide to the hybridization of nucleic acids is found in Tijssen (1993) Laboratory Techniques in Biochemistry and Molecular Biology-Hybridization with Nucleic Acid Probes part I chapter 2 "Overview of principles of hybridization and the strategy of nucleic acid probe assays" Elsevier, New York. Generally, highly stringent hybridization and wash conditions are selected to be about 5.degree. C. lower than the thermal melting point (T.sub.m) for the specific sequence at a defined ionic strength and pH. Typically, under "stringent conditions" a probe will hybridize to its target subsequence, but to no other sequences.

The T.sub.m is the temperature (under defined ionic strength and pH) at which 50% of the target sequence hybridizes to a perfectly matched probe. Very stringent conditions are selected to be equal to the T.sub.m for a particular probe. An example of stringent hybridization conditions for hybridization of complementary nucleic acids which have more than 100 complementary residues on a filter in a Southern or northern blot is 50% formamide with 1 mg of heparin at 42.degree. C., with the hybridization being carried out overnight. An example of highly stringent wash conditions is 0.1 5M NaCl at 72.degree. C. for about 15 minutes. An example of stringent wash conditions is a 0.2.times.SSC wash at 65.degree. C. for 15 minutes (see, Sambrook, infra, for a description of SSC buffer). Often, a high stringency wash is preceded by a low stringency wash to remove background probe signal. An example medium stringency wash for a duplex of, e.g., more than 100 nucleotides, is 1.times.SSC at 45.degree. C. for 15 minutes. An example low stringency wash for a duplex of, e.g., more than 100 nucleotides, is 4-6.times.SSC at 40.degree. C. for 15 minutes. For short probes (e.g., about 10 to 50 nucleotides), stringent conditions typically involve salt concentrations of less than about 1.0M Na ion, typically about 0.01 to 1.0 M Na ion concentration (or other salts) at pH 7.0 to 8.3, and the temperature is typically at least about 30.degree. C. Stringent conditions can also be achieved with the addition of destabilizing agents such as formamide. In general, a signal to noise ratio of 2.times. (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

A "plant" is any plant at any stage of development, particularly a seed plant.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, zygotes and embryos at various stages of development.

"Plant material" refers to leaves, stems, roots, flowers or flower parts, fruits, pollen, egg cells, zygotes, seeds, cuttings, cell or tissue cultures, or any other part or product of a plant.

A "plant organ" is a distinct and visibly structured and differentiated part of a plant such as a root, stem, leaf, flower bud, or embryo.

"Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

In one embodiment, the invention relates to a maize plant, which plant has a genome comprising a set of alleles associated with a corresponding set of QTLs of economic importance and genetically linked to the markers as shown in Table A-G, wherein said set of QTLs comprises at least two QTLs, particularly at least 5, more particularly at least 10, even more particularly at least 20 and up to 37 QTLs contributing to a phenotypic trait selected from the group of grain yield, grain moisture at harvest, early and late root lodging, stalk lodging, common smut incidence, fusarium ear rot incidence, sulcotrione resistance, and tassel architecture.

In particular, the invention relates to a maize plant containing a nuclear genome comprising a set of alleles at a corresponding set of QTLs each of which contribute to a phenotypic trait of economic importance, wherein
a) each QTL is genetically linked to at least one marker locus, which can be identified by a pair of PCR oligonucleotide primers consisting of a forward primer and a reverse primer exhibiting a nucleotide sequence as given in SEQ ID NO: 1 - 82 shown in Tables A-G; and
b) each allele at the corresponding QTL is defined by at least one marker allele at said at least one marker locus linked to the QTL, which marker allele is characterized by the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair; and wherein said
set of QTLs comprises at least 10, particularly at least 15, more particularly at least 20, even more particularly at least 25, but especially at least 30 and up to 37 different QTLs.

The primer pairs recited above in steps a) and b) are comprised of a forward primer with an odd-numbered sequence identification number and a reverse primer with the next higher even-numbered sequence identification number. For example, forward primer with SEQ ID NO: 1 and reverse primer with SEQ ID NO: 2 are building a primer pair, as do SEQ ID NO: 3 and SEQ ID NO: 4; SEQ ID NO: 5 and SEQ ID NO: 6, etc.

The PCR amplification product recited above in steps b) obtained in a PCR reaction with an oligonucleotide primer pair given in Tables A-G, can be identified based on its molecular weight or nucleotide sequence, both of which are essentially identical to the molecular weight or nucleotide sequence of the corresponding PCR amplification product of the favourable allele as indicated in Tables A-G obtainable from inbred lines M3047/1 (NCIMB 41459) and M3047/2 (NCIMB 41460) in a PCR reaction with the identical primer pair.

In a specific embodiment, said maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A, wherein said set of QTLs comprises at least 5 particularly at least 8, more particularly at least 10, even more particularly at least 14, different QTLs contributing to the phenotypic trait of grain yield, which QTLs are mapping to loci on chromosomes 1, 2, 4, 5, and 7, wherein each allele at the corresponding QTL is defined by at least one marker allele at said at least one marker locus linked to the QTL, which marker allele is characterized by the PCR amplification product of the respective oligonucleotide primer pair given in Table A, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In one aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A, wherein said set of QTLs comprises 14 different QTLs contributing to the phenotypic trait of grain yield, which QTLs are mapping to loci on chromosomes 1, 2, 4, 5, and 7, wherein each allele at the corresponding QTL is defined by at least one marker allele at said at least one marker locus linked to the QTL, which marker allele is characterized by the PCR amplification product of the respective oligonucleotide primer pair given in Table A, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another specific embodiment, said maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, wherein said set of QTLs comprises at least 5 particularly at least 7, more particularly at least 9, even more particularly at least 11, different QTLs contributing to the phenotypic trait of grain moisture at harvest, which QTLs are mapping to loci on chromosomes 1, 2, 3, 4, 5, 7 and 8, wherein each allele at the corresponding QTL is defined by at least one marker allele at said at least one marker locus linked to the QTL, which marker allele is characterized by the PCR amplification product of the respective oligonucleotide primer pair given in Table B, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In one aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, wherein said set of QTLs comprises 11 different QTLs contributing to the phenotypic trait of grain moisture at harvest, which QTLs are mapping to loci on chromosomes 1, 2, 4, 5, and 7, wherein each allele at the corresponding QTL is defined by at least one marker allele at said at least one marker locus linked to the QTL, which marker allele is characterized by the PCR amplification product of the respective oligonucleotide primer pair given in Table B, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In still another specific embodiment, said maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3, 4, 27, 28, 45-48, 59 and 60 shown in Table C, wherein said set of QTLs comprises at least 4 different QTLs, but particularly 3 QTLs, contributing to the phenotypic trait of early and late root lodging/stalk lodging, which QTLs are mapping to loci on chromosomes 1, wherein each allele at the corresponding QTL is defined by at least one marker allele at said at least one marker locus linked to the QTL, which marker allele is characterized by the PCR amplification product of the respective oligonucleotide primer pair given in Table C, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In still another specific embodiment, said maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 7, 8, 11, 12, 31, 32, 39, 40, 55, 56, 81 and 82 shown in Table E, wherein said set of QTLs comprises at least 4 different QTLs, but particularly 4 QTLs, contributing to the phenotypic trait of tassel architecture, which QTLs are mapping to loci on chromosomes 3, 6, 7 and 9, wherein each allele at the corresponding QTL is defined by at least one marker allele at said at least one marker locus linked to the QTL, which marker allele is characterized by the PCR amplification product of the respective oligonucleotide primer pair given in Table E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In still another specific embodiment, said maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 11 and 12 shown in Table D, as given in SEQ ID NO: 7, 8, 43, 44, 81 and 82 shown in Table F and as given in SEQ ID NO: 1, 2, 15, 16, 79 and 80 shown in Table G, respectively, wherein said set of QTLs comprises at least 1, particularly at least 2, more particularly at least 4 different QTLs contributing to the phenotypic trait of fungal resistance or incidence selected from the group consisting of sulcotrione resistance, fusarium incidence and common smut incidence, which QTLs are mapping to loci on chromosomes 3, 5 and 9, wherein each allele at the corresponding QTL is defined by at least one marker allele at said at least one marker locus linked to the QTL, which marker allele is characterized by the PCR amplification product of the respective oligonucleotide primer pair given in Table D, F and G, respectively, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In one aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer as given in SEQ ID NO: 11 and 12 shown in Table D, as given in SEQ ID NO: 7, 8, 43, 44, 81 and 82 shown in Table F and as given in SEQ ID NO: 1, 2, 15, 16, 79 and 80 shown in Table G, respectively, wherein said set of QTLs comprises 2 different QTLs contributing to the phenotypic trait of fusarium ear-rot incidence, which QTLs are mapping to loci on chromosome 5, 2 different QTLs contributing to the phenotypic trait of sulcotrione resistance mapping to loci on chromosomes 3 and 9, and 1 QTL contributing to the phenotypic trait of common smut incidence mapping to a locus on chromosome 3, wherein each allele at the corresponding QTL is defined by at least one marker allele at said at least one marker locus linked to the QTL, which marker allele is characterized by the PCR amplification product of the respective oligonucleotide primer pair given in Table D, F and G, respectively, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific embodiment, said maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A and as given in SEQ ID NO: 3-6, 9, 10, 13, 14, 21-24, 29-34, 37-40, 43, 44, 53, 54, 57, 58 and 65-72 shown in Table B, wherein said set of QTLs comprises at least 10, particularly at least 15, more particularly at least 20, even more particularly at least 25, different QTLs contributing to the phenotypic trait of grain yield and grain moisture at harvest, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables A and B, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A and as given in SEQ ID NO: 3-6, 9, 10, 13, 14, 21-24, 29-34, 37-40, 43, 44, 53, 54, 57, 58 and 65-72 shown in Table B, wherein said set of QTLs comprises 25 different QTLs, 14 of which are contributing to grain yield and mapping to loci on chromosome 1, 2, 4, 5 and 7 and 11 QTLs are contributing to grain moisture mapping to loci on chromosome 1, 2, 3, 4, 5, 7 and 8, wherein each allele at the corresponding QTL is defined by at least one marker allele at said at least one marker locus linked to the QTL, which marker allele is characterized by the PCR amplification product of the respective oligonucleotide primer pair given in Tables A and B, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In still another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B and as given in SEQ ID NO: 3, 4, 27, 28, 45-48, 59 and 60 shown in Table C, wherein said set of QTLs comprises at least 10, particularly at least 15, more particularly at least 20, even more particularly at least 25, but especially at least 28 different QTLs contributing to the phenotypic trait of grain yield, grain moisture at harvest and early and late root lodging, stalk lodging, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables A-C, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In on aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B and as given in SEQ ID NO: 3, 4, 27, 28, 45-48, 59 and 60 shown in Table C, wherein said set of QTLs comprises 28 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, and 3 QTLs are contributing to root and stalk lodging and are mapping to chromosome 1, wherein each allele at the corresponding QTL is defined by at least one marker allele at said at least one marker locus linked to the QTL, which marker allele is characterized by the PCR amplification product of the respective oligonucleotide primer pair given in Tables A-C, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C and as given in SEQ ID NO: 11 and 12 shown in Table D, wherein the set of QTLs comprises at least 10, particularly at least 15, more particularly at least 20, even more particularly at least 25, but especially at least 29 different QTLs contributing to the phenotypic trait of grain yield, grain moisture at harvest, early and late root lodging, stalk lodging and common smut incidence, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables A-D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In particular, the invention provides a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C and as given in SEQ ID NO: 11 and 12 shown in Table D, wherein the set of QTLs comprises 29 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, and 1 QTL is contributing to common smut incidence and is mapping to a locus on chromosome 3, wherein each allele at the corresponding QTL is defined by at least one marker allele at said at least one marker locus linked to the QTL, which marker allele is characterized by the PCR amplification product of the respective oligonucleotide primer pair given in Tables A-D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, and as given in SEQ ID NO: 11 and 12 shown in Table D, wherein the set of QTLs comprises at least 10, particularly at least 15, more particularly at least 20, even more particularly at least 25, but especially at least 26 different QTLs contributing to the phenotypic trait of grain yield, grain moisture at harvest, and common smut incidence, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables A, B and D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In particular, the invention provides a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, and as given in SEQ ID NO: 11 and 12 shown in Table D, wherein the set of QTLs comprises 26 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, and 1 QTL is contributing to common smut incidence and is mapping to a locus on chromosome 3, wherein each allele at the corresponding QTL is defined by at least one marker allele at said at least one marker locus linked to the QTL, which marker allele is characterized by the PCR amplification product of the respective oligonucleotide primer pair given in Tables A,B, and D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C and as given in SEQ ID NO: 11 and 12 shown in Table D, wherein the set of QTLs comprises at least 8, particularly at least 12, more particularly at least 15, but especially at least 18 different QTLs contributing to the phenotypic trait of grain yield, late root lodging, stalk lodging and common smut incidence, which QTLs are mapping to loci on chromosomes 1, 2, 3, 4, 5, and 7, wherein each allele at the corresponding QTL is defined by at least one marker allele at said at least one marker locus linked to the QTL, which marker allele is characterized by the PCR amplification product of the respective oligonucleotide primer pair given in Tables A, C and D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In particular, the invention provides a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C and as given in SEQ ID NO: 11 and 12 shown in Table D, wherein the set of QTLs comprises 18 different QTLs, 14 of which are contributing to grain yield and are mapping to loci on chromosome 1, 2, 4, 5 and 7; 3 QTLs are contributing to root and stalk lodging and are mapping to chromosome 1, and 1 QTL is contributing to common smut incidence and is mapping to a locus on chromosome 3, wherein each allele at the corresponding QTL is defined by at least one marker allele at said at least one marker locus linked to the QTL, which marker allele is characterized by the PCR amplification product of the respective oligonucleotide primer pair given in Tables A, C and D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C and as given in SEQ ID NO: 11 and 12 shown in Table D, wherein the set of QTLs comprises at least 8, particularly at least 12, more particularly at least 15, but especially at least 15 different QTLs contributing to the phenotypic trait of grain moisture at harvest, early and late root lodging, stalk lodging and common smut incidence, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables B, C and D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In particular, the invention provides a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C and as given in SEQ ID NO: 11 and 12 shown in Table D, wherein the set of QTLs comprises 15 different QTLs, 11 QTLs are contributing to grain moisture and are mapping to loci on chromosome 1, 2, 3, 4, 5, 7 and 8, 3 QTLs are contributing to root and stalk lodging and are mapping to chromosome 1, and 1 QTL is contributing to common smut incidence and is mapping to a locus on chromosome 3, wherein each allele at the corresponding QTL is defined by at least one marker allele at said at least one marker locus linked to the QTL, which marker allele is characterized by the PCR amplification product of the respective oligonucleotide primer pair given in Tables B, C and D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair..

In other embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises at least 10, particularly at least 15, more particularly at least 20, even more particularly at least 25, but especially at least 30 and up to 33 different QTLs contributing to the phenotypic trait of grain yield, grain moisture at harvest and early, late root lodging, stalk lodging, common smut incidence and tassel architecture, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises 33 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3 and 4 QTLs are contributing to tassel architecture and are mapping to loci on chromosomes 3, 6, 7 and 9, wherein each allele at the corresponding QTL is defined by at least one marker allele at said at least one marker locus linked to the QTL, which marker allele is characterized by the PCR amplification product of the respective oligonucleotide primer pair given in Tables A-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises at least 8, particularly at least 12, more particularly at least 15, but especially at least 19 different QTLs contributing to the phenotypic trait of grain moisture at harvest and early, late root lodging, stalk lodging, common smut incidence and tassel architecture, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables B-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises 19 different QTLs, with 11 QTLs contributing to grain moisture and are mapping to loci on chromosome 1, 2, 3, 4, 5, 7 and 8, 3 QTLs contributing to root and stalk lodging and are mapping to chromosome 1 and 5, 1 QTL contributing to common smut incidence and is mapping to a locus on chromosome 3 and 4 QTLs contributing to tassel architecture and are mapping to loci on chromosomes 3, 6, 7 and 9, wherein each allele at the corresponding QTL is defined by at least one marker allele at said at least one marker locus linked to the QTL, which marker allele is characterized by the PCR amplification product of the respective oligonucleotide primer pair given in Tables B-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E Table, wherein the set of QTLs comprises at least 10, particularly at least 15, more particularly at least 20, but especially at least 22 different QTLs contributing to the phenotypic trait of grain yield, early, late root lodging, stalk lodging, common smut incidence and tassel architecture, which QTLs are mapping to loci on chromosomes 1, 2, 3, 4, 5, 6, 7, and 9, wherein each allele at the corresponding QTL is defined by at least one marker allele at said at least one marker locus linked to the QTL, which marker allele is characterized by the PCR amplification product of the respective oligonucleotide primer pair given in Tables A, and C-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises 22 different QTLs, 14 of which are contributing to grain yield and are mapping to loci on chromosome 1, 2, 4, 5 and 7; 3 QTLs are contributing to root and stalk lodging and are mapping to chromosome 1, 1 QTL is contributing to common smut incidence and is mapping to a locus on chromosome 3 and 4 QTLs are contributing to tassel architecture and are mapping to loci on chromosomes 3, 6, 7 and 9, wherein each allele at the corresponding QTL is defined by at least one marker allele at said at least one marker locus linked to the QTL, which marker allele is characterized by the PCR amplification product of the respective oligonucleotide primer pair given in Table A and C-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises at least 10, particularly at least 15, more particularly at least 20, even more particularly at least 25, but especially at least 30 different QTLs contributing to the phenotypic trait of grain yield, grain moisture at harvest, common smut incidence and tassel architecture, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A, B, D and E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises 30 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 1 QTL is contributing to common smut incidence and is mapping to a locus on chromosome 3 and 4 QTLs are contributing to tassel architecture and are mapping to loci on chromosomes 3, 6, 7 and 9, wherein each allele at the corresponding QTL is defined by at least one marker allele at said at least one marker locus linked to the QTL, which marker allele is characterized by the PCR amplification product of the respective oligonucleotide primer pair given in Tables A, B, D and E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, and as given in SEQ ID NO: 7, 8, 11, 12, 31, 32, 39, 40, 55, 56, 81 and 82 shown in Table E, wherein the set of QTLs comprises at least 10, particularly at least 15, more particularly at least 20, even more particularly at least 25, but especially at least 30 and up to 32 different QTLs contributing to the phenotypic trait of grain yield, grain moisture at harvest and early, late root lodging, stalk lodging, and tassel architecture, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-C and E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, and as given in SEQ ID NO: 7, 8, 11, 12, 31, 32, 39, 40, 55, 56, 81 and 82 shown in Table E, wherein the set of QTLs comprises 32 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, and 4 QTLs are contributing to tassel architecture and are mapping to loci on chromosomes 3, 6, 7 and 9, wherein each allele at the corresponding QTL is defined by at least one marker allele at said at least one marker locus linked to the QTL, which marker allele is characterized by the PCR amplification product of the respective oligonucleotide primer pair given in Tables A-C and E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises at least 10, particularly at least 15, more particularly at least 20, even more particularly at least 25, but especially at least 30 and up to 35 different QTLs contributing to the phenotypic trait of grain yield, grain moisture at harvest and early, late root lodging, stalk lodging, common smut incidence, tassel architecture and sulcotrione resistance, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises 35 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is characterized by the PCR amplification product of the respective oligonucleotide primer pair as shown pintables A-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises at least 10, particularly at least 15, more particularly at least 20, but especially at least 21 different QTLs contributing to the phenotypic trait of grain moisture at harvest and early, late root lodging, stalk lodging, common smut incidence, tassel architecture and sulcotrione resistance, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables B-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises 21 different QTLs, 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is characterized by the PCR amplification product of the respective oligonucleotide primer pair given in Tables B-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises at least 10, particularly at least 15, more particularly at least 20, but especially at least 24 different QTLs contributing to the phenotypic trait of grain yield, early, late root lodging, stalk lodging, common smut incidence, tassel architecture and sulcotrione resistance, which QTLs are mapping to loci on chromosomes 1, 2, 3, 4, 5, 6, 7, and 9, wherein each allele at the corresponding QTL is defined by at least one marker allele at said at least one marker locus linked to the QTL, which marker allele is characterized by the PCR amplification product of the respective oligonucleotide primer pair given in Tables A and C-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises 24 different QTLs, 14 of which are contributing to grain yield and are mapping to loci on chromosome 1, 2, 4, 5 and 7; 3 QTLs are contributing to root and stalk lodging and are mapping to chromosome 1 and 5, 1 QTL is contributing to common smut incidence and is mapping to a locus on chromosome 3, 4 QTLs are contributing to tassel architecture and are mapping to loci on chromosomes 3, 6, 7 and 9, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is characterized by the PCR amplification product of the respective oligonucleotide primer pair given in Tables A and C-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises at least 10, particularly at least 15, more particularly at least 20, even more particularly at least 25, but especially at least 30 and up to 32 different QTLs contributing to the phenotypic trait of grain yield, grain moisture at harvest, common smut incidence, tassel architecture and sulcotrione resistance, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A, B and D-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises 32 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 1 QTL is contributing to common smut incidence and is mapping to a locus on chromosome 3, 4 QTLs are contributing to tassel architecture and are mapping to loci on chromosomes 3, 6, 7 and 9, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is characterized by the PCR amplification product of the respective oligonucleotide primer pair given in Tables A, B and D-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises at least 10, particularly at least 15, more particularly at least 20, even more particularly at least 25, but especially at least 30 and up to 34 different QTLs contributing to the phenotypic trait of grain yield, grain moisture at harvest, late root lodging, stalk lodging, tassel architecture and sulcotrione resistance, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-C, E and F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises 34 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 4 QTLs are contributing to tassel architecture and are mapping to loci on chromosomes 3, 6, 7 and 9, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is characterized by the PCR amplification product of the respective oligonucleotide primer pair given in Tables A-C, E and F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 43, 44, 81 and 82 shown in Table F, wherein the set of QTLs comprises at least 10, particularly at least 15, more particularly at least 20, even more particularly at least 25, but especially at least 30 and up to 31 different QTLs contributing to the phenotypic trait of grain yield, grain moisture at harvest and early, late root lodging, stalk lodging, common smut incidence, and sulcotrione resistance, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables A-D and F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 43, 44, 81 and 82 shown in Table F, wherein the set of QTLs comprises 31 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is characterized by the PCR amplification product of the respective oligonucleotide primer pair given in Tables A-D and F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises at least 10, particularly at least 15, more particularly at least 20, even more particularly at least 25, but especially at least 30 and up to 37 different QTLs contributing to the phenotypic trait of grain yield, grain moisture at harvest, late root lodging, stalk lodging, common smut incidence, tassel architecture, sulcotrione resistance and fusarium ear rot incidence, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 37 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises at least 10, particularly at least 15, more particularly at least 20, but especially at least 23 different QTLs contributing to the phenotypic trait of grain moisture at harvest and early, late root lodging, stalk lodging, common smut incidence, tassel architecture, sulcotrione resistance and fusarium ear rot incidence, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables B-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 23 different QTLs , 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables B-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises at least 10, particularly at least 15, more particularly at least 20, but especially at least 25 and up to 26 different QTLs contributing to the phenotypic trait of grain yield, late root lodging, stalk lodging, common smut incidence, tassel architecture, sulcotrione resistance and fusarium ear rot incidence, which QTLs are mapping to loci on chromosomes 1, 2, 3, 4, 5, 6, 7, and 9, wherein each allele at the corresponding QTL is defined by at least one marker allele at said at least one marker locus linked to the QTL, which marker allele is characterized by the PCR amplification product of the respective oligonucleotide primer pair given in Tables A and C-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 26 different QTLs, 14 of which are contributing to grain yield and are mapping to loci on chromosome 1, 2, 4, 5 and 7; 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables A and C-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises at least 10, particularly at least 15, more particularly at least 20, even more particularly at least 25, but especially at least 30 and up to 34 different QTLs contributing to the phenotypic trait of grain yield, grain moisture at harvest, common smut incidence, tassel architecture, sulcotrione resistance and fusarium ear rot incidence, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A, B and D-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 34 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 1 QTL is contributing to common smut incidence and is mapping to a locus on chromosome 3, 4 QTLs are contributing to tassel architecture and are mapping to loci on chromosomes 3, 6, 7 and 9, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables A, B and D-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises at least 10, particularly at least 15, more particularly at least 20, even more particularly at least 25, but especially at least 30 and up to 36 different QTLs contributing to the phenotypic trait of grain yield, grain moisture at harvest and early, late root lodging, stalk lodging, tassel architecture, sulcotrione resistance and fusarium ear rot incidence, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-C and E-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 36 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 4 QTLs are contributing to tassel architecture and are mapping to loci on chromosomes 3, 6, 7 and 9, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables A-C and E-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles associated with a corresponding set of QTLs and genetically-linked to the markers as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises at least 10, particularly at least 15, more particularly at least 20, even more particularly at least 25, but especially at least 30 and up to 33 different QTLs contributing to the phenotypic trait of grain yield, grain moisture at harvest and early, late root lodging, stalk lodging, common smut incidence, sulcotrione resistance and fusarium ear rot incidence, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is characterized by the PCR amplification product of the respective oligonucleotide primer pair given in Tables A-D, F and G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 33 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables A-D, F and G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In another embodiment of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 1, 2, 15, 16, 79 and 80 shown in Table G, wherein the set of QTLs comprises at least 10, particularly at least 15, more particularly at least 20, even more particularly at least 25, but especially at least 30 and up to 35 different QTLs contributing to the phenotypic trait of grain yield, grain moisture at harvest and early, late root lodging, stalk lodging, common smut incidence, tassel architecture, and fusarium ear rot incidence, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is characterized by the PCR amplification product of the respective oligonucleotide primer pair given in Tables A-E and G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific aspect of the invention, a maize plant according to the invention and described herein before is characterized by a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 1, 2, 15, 16, 79 and 80 shown in Table G, wherein the set of QTLs comprises 35 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is characterized by the PCR amplification product of the respective oligonucleotide primer pair given in Tables A-E and G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

In a specific embodiment, the invention relates to a maize plant containing a nuclear genome comprising a set of alleles at a corresponding set of QTLs each of which contributes to a phenotypic trait selected from the group of grain yield, grain moisture at harvest, early and late root lodging, stalk lodging, common smut incidence, fusarium ear rot incidence, sulcotrione resistance, and tassel architecture, wherein
a) each QTL is genetically linked to at least one marker locus, which can be identified by a pair of PCR oligonucleotide primers consisting of a forward primer and a reverse primer exhibiting a nucleotide sequence as given in SEQ ID NO: 1 - 82 shown in Tables A-G; and
b) each allele at the corresponding QTL is defined by at least one marker allele at said at least one marker locus linked to the QTL, which marker allele is characterized by the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair, and wherein said
set of QTLs comprises 37 different QTLs as given in Tables A-G, but particularly a maize plant wherein at least part of said QTLs are obtained from maize inbred lines M3047/2 (NCIMB 41460) and M3047/1 (NCIMB 41459)), respectively.

In one embodiment, the plant according to the invention and as described herein before is an inbred.

In another embodiment, the plant according to the invention and as described herein before is a hybrid.

The present invention also contemplates improved inbred and hybrid maize plants, and progeny thereof, which have introgressed into its genome, genetic material from at least one, preferably more than one, and most preferably all, of the hereinbefore described quantitative trait loci, particularly improved inbred and hybrid maize plants, and progeny thereof, which exhibit the traits of high grain yield and low grain moisture at harvest.

In a specific embodiment of the invention, a maize plant is provided as described herein before, wherein said plant always carries the most favourable allele at the marker loci linked to the QTL.

In particular, the invention relates to a maize plant as described herein before, wherein said favorable allele is in the homozygous state.

In still another specific embodiment a maize plant is provided according to the invention and as described herein before which maize plant carries the most favourable allele at the marker loci linked to the QTL shown in Tables A-G.

In one aspect, the invention relates to a marker or a set of two or more markers and up to 41 markers comprising a pair of PCR oligonucleotide primers consisting of a forward primer and a reverse primer exhibiting a nucleotide sequence as given in SEQ ID NO: 1-82 shown in Tables A-G, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In another aspect, the invention relates to a marker or a set of two or more markers and up to 20 markers comprising a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In still another aspect, the invention relates to a marker or a set of two or more markers and up to 18 markers comprising a pair of PCR oligonucleotide primers consisting of a forward primer and a reverse primer exhibiting a nucleotide sequence as given in SEQ ID NO: 3-6, 9, 10, 13, 14, 21-24, 29-34, 37-40, 43, 44, 53, 54, 57, 58 and 65-72 shown in Table B, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In still another aspect, the invention relates to a marker or a set of two or more markers and up to 41 markers comprising a pair of PCR oligonucleotide primers consisting of a forward primer and a reverse primer exhibiting a nucleotide sequence as given in SEQ ID NO: 3, 4, 27, 28, 45-48, 59 and 60 shown in Table C, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In still another aspect, the invention relates to a marker or a set of two or more markers and up to 5 markers comprising a pair of PCR oligonucleotide primers consisting of a forward primer and a reverse primer exhibiting a nucleotide sequence as given in SEQ ID NO: 11 and 12 shown in Table D, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In still another aspect, the invention relates to a marker or a set of two or more markers and up to 6 markers comprising a pair of PCR oligonucleotide primers consisting of a forward primer and a reverse primer exhibiting a nucleotide sequence as given in SEQ ID NO: 7, 8, 11, 12, 31, 32, 39, 40, 55, 56, 81 and 82 shown in Table E, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In still another aspect, the invention relates to a marker or a set of two or more markers and up to 3 markers comprising a pair of PCR oligonucleotide primers consisting of a forward primer and a reverse primer exhibiting a nucleotide sequence as given in SEQ ID NO: 7, 8, 43, 44, 81 and 82 shown in Table F, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In still another aspect, the invention relates to a marker or a set of two or more markers and up to 3 markers comprising a pair of PCR oligonucleotide primers consisting of a forward primer and a reverse primer exhibiting a nucleotide sequence as given in SEQ ID NO: 1, 2, 15, 16, 79 and 80 shown in Table G, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In a specific embodiment, the invention relates to a set of markers which can be chosen from Tables A-G and compiled such that they are capable of detecting any one of the different sub-groups of alleles identified herein before.

The primer pairs according to the invention and described herein before to be used in a PCR amplification reaction for amplifying a DNA fragment which is characteristic of the marker allele according to the invention, are comprised of a forward primer with an odd-numbered sequence identification number and a reverse primer with the next higher even-numbered sequence identification number. For example, forward primer with SEQ ID NO: 1 and reverse primer with SEQ ID NO: 2 are building a primer pair, as do SEQ ID NO: 3 and SEQ ID NO: 4; SEQ ID NO: 5 and SEQ ID NO: 6, etc.

The conditions used in the PCR amplification reaction are standard conditions well known to those skilled in the art involving PCR buffer and salt solutions, dNPs, an appropriate polymerase, particularly a Taq polymerase and the appropriate forward and reverse primers in suitable concentrations.

The PCR amplification comprises between 20 and 100 amplification cycles, particularly between 30 and 80 amplification cycles, more particularly between 40 and 60 amplification cycles, but especially 40 amplification cycles of between 40 sec to 5 minutes, particularly between 50 sec and 2 minutes, more particularly between 60 sec and 90 sec, but especially 60 sec.

Within such an amplification cycle the DNA is first subjected to heat in the range of between 90°C and 98°C, particularly between 92°C and 96°C, but especially 94°C for between 5 sec and 30 sec, particularly for between 10 sec and 20 sec, but especially for 15 sec. The process is continued at a temperature of between 35°C and 65°C, particularly between 40°C and 60°C, but especially at 59°C, optionally followed by an incubation of the DNA for between 1 and 5 minutes, particularly for between 2 and 3 minutes, but especially for 2 minutes at a temperature of between 65°C and 80°C, particularly between 70°C and 75°C, but especially at 72°C.

The PCR amplification according to the invention and described herein before, which are obtained in a PCR reaction with an oligonucleotide primer pair given in any one of Tables A-G, can be identified based on its molecular weight or nucleotide sequence, both of which are essentially identical to the molecular weight or nucleotide sequence of the corresponding PCR amplification product obtainable from inbred lines M3047/1 (NCIMB 41459) and M3047/2 (NCIMB 41460) in a PCR reaction with the identical primer pair.

In one embodiment, the invention relates to plant material obtainable from a plant according to the invention and as described herein before including, but without being limited thereto, leaves, stems, roots, flowers or flower parts, fruits, pollen, egg cells, zygotes, seeds, cuttings, cell or tissue cultures, or any other part or product of the plant. The invention further relates to plant parts obtainable from a plant according to the invention and as described herein before including, but without being limited thereto, plant seed, plant organs such as, for example, a root, stem, leaf, flower bud, or embryo, etc, ovules, pollen microspores, plant cells, plant tissue, plant cells cultures such as, for example, protoplasts, cell culture cells, cells in plant tissues, pollen, pollen tubes, ovules, embryo sacs, zygotes and embryos at various stages of development, etc.

The invention also relates to processed maize products particularly products resulting from wet or dry milling of maize grains including, without being limited thereto, grinded grains, flour, oil cake, fermented products, etc, further to kernels or grains to be used in animal feed formulations processed through, for example, kernel cracking or steam flaking.

In one embodiment, the invention relates to a method of producing a plant according to the present invention and as disclosed herein before comprising the steps of
i) crossing two or more parent plants which have a genetic background capable of contributing to the development of a plant according to the invention and as described herein before, particularly crossing two parent plants which have a genetic background as represented by maize inbred lines M3047/1 (NCIMB 41459) and M3047/2 (NCIMB 41460), or an ancestor or progenitor plant thereof,
ii) screening for a plant which has in its genome a set of alleles at a corresponding set of QTLs, with each QTL being genetically-linked to at least one marker locus, wherein said set of QTLs comprises at least 10, particularly at least 15, more particularly at least 20, even more particularly at least 25, but especially at least 30 and up to 37 different QTLs, wherein each allele at the corresponding QTL is defined by at least one marker allele at said at least one marker locus linked to the QTL by
   1. identifying the at least one marker locus in a PCR reaction using a pair of PCR oligonucleotide primers consisting of a forward primer and a reverse primer exhibiting a nucleotide sequence as given in SEQ ID NO: 1-82 shown in Table A-G, and
   2. identifying the marker allele by determining the molecular weight of the PCR amplification product obtained in step 1.
iii) selecting a plant with the desired profile.

In particular, the invention relates to a method wherein at least one of the parental plants has a genome comprising a sub-set of alleles which are associated with a corresponding sub-set of QTLs genetically-linked to a marker locus which can be identified in a PCR reaction using a pair of PCR oligonucleotide primers consisting of a forward primer and a reverse primer exhibiting a nucleotide sequence as given in SEQ ID NO: 1-82 shown in Table A-G, wherein said sub-set of QTLs comprises at least two QTLs, particularly at least 5, more particularly at least 10, even more particularly at least 15, but especially 20 and up to 30-37 QTLs contributing to a phenotypic trait selected from the group of grain yield, grain moisture at harvest, early and late root lodging, stalk lodging, common smut incidence, fusarium ear rot incidence, sulcotrione resistance, and tassel architecture.

In a specific embodiment, the invention relates to a method wherein at least one of the parental plants has a genome comprising a sub-set of alleles which are associated with a corresponding sub-set of QTLs genetically-linked to a marker locus which can be identified in a PCR reaction using a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A, wherein said sub-set of QTLs comprises at least 5 particularly at least 8, more particularly at least 10, even more particularly at least 14, different QTLs contributing to the phenotypic trait of grain yield, which QTLs are mapping to loci on chromosomes 1, 2, 4, 5, and 7.

In another specific embodiment, the invention relates to a method wherein at least one of the parental plants has a genome comprising a sub-set of alleles which are associated with a corresponding sub-set of QTLs genetically-linked to as given in SEQ ID NO: 3-6, 9, 10, 13, 14, 21-24, 29-34, 37-40, 43, 44, 53, 54, 57, 58 and 65-72 shown in Table B, wherein said sub-set of QTLs comprises at least 5 particularly at least 7, more particularly at least 9, even more particularly at least 11, different QTLs contributing to the phenotypic trait of grain moisture at harvest, which QTLs are mapping to loci on chromosomes 1, 2, 3, 4, 5, 7 and 8.

In still another specific embodiment, the invention relates to a method wherein at least one of the parental plants has a genome comprising a sub-set of alleles which are associated with a corresponding sub-set of QTLs genetically-linked to as given in SEQ ID NO: 3, 4, 27, 28, 45-48, 59 and 60 shown in Table C, wherein said sub-set of QTLs comprises at least 1, particularly at least 2, more particularly at least 3, but especially at least 4 different QTLs contributing to the phenotypic trait of early and late root lodging/stalk lodging, which QTLs are mapping to loci on chromosomes 1, and 5.

In still another specific embodiment, the invention relates to a method wherein at least one of the parental plants has a genome comprising a sub-set of alleles which are associated with a corresponding sub-set of QTLs genetically-linked to as given in SEQ ID NO: 7, 8, 11, 12, 31, 32, 39, 40, 55, 56, 81 and 82 shown in Table E, wherein said sub-set of QTLs comprises at least 1, particularly at least 2, more particularly at least 3, but especially at least 4 different QTLs contributing to the phenotypic trait of tassel architecture, which QTLs are mapping to loci on chromosomes 3, 6, 7 and 9.

In still another specific embodiment, the invention relates to a method wherein at least one of the parental plants has a genome comprising a sub-set of alleles which are associated with a corresponding sub-set of QTLs genetically-linked to as given in SEQ ID NO: 11 and 12 shown in Table D, as given in SEQ ID NO: 7, 8, 43, 44, 81 and 82 shown in Table F and as given in SEQ ID NO: 1, 2, 15, 16, 79 and 80 shown in Table G, wherein said sub-set of QTLs comprises at least 1, particularly at least 2, more particularly at least 4 different QTLs contributing to the phenotypic trait of fungal resistance or incidence selected from the group consisting of sulcotrione resistance, fusarium ear rot incidence and common smut incidence, which QTLs are mapping to loci on chromosomes 3, 5 and 9.

In one embodiment, the invention relates to a method wherein at least one of the parental plants has a genome comprising any one of the sub-sets of alleles at a corresponding set of QTLs as defined herein before.

In a specific embodiment, the invention relates to a method for producing a hybrid maize plant according to the present invention and as disclosed herein before comprising the steps of
i) crossing an inbred plant according to the invention and as disclosed herein before with a maize inbred line exhibiting desirable properties which take effect through phenotypically detectable traits to produce a segregating population of plants,
ii) screening the plants within this segregating population for the presence of a plant which has in its genome a set of alleles at a corresponding set of QTLs, with each QTL being genetically-linked to at least one marker locus, wherein said set of QTLs comprises at least 10, particularly at least 15, more particularly at least 20, even more particularly at least 25, but especially at least 30 and up to 37 different QTLs, wherein each allele at the corresponding QTL is defined by at least one marker allele at said at least one marker locus linked to the QTL by
   a. identifying the at least one marker locus in a PCR reaction using a pair of PCR oligonucleotide primers consisting of a forward primer and a reverse primer exhibiting a nucleotide sequence as given in SEQ ID NO: 1-82 shown in Table A-G, and
   b. identifying the marker allele by determining the molecular weight of the PCR amplification product obtained in step a).
iii) selecting hybrid plants from the segregating population with the QTL profile indicated step ii) above.

In particular, the invention relates to a single cross F₁ hybrid.

In one embodiment, the invention relates to a method of using a set of nucleic acid markers in marker-based selection for introgressing a set of alleles which are associated to a corresponding set of QTLs into maize germplasm lacking said set of alleles, wherein said alleles contribute to a phenotypic trait selected from the group of grain yield, grain moisture at harvest, early and late root lodging, stalk lodging, common smut incidence, fusarium ear rot incidence, sulcotrione resistance, and tassel architecture and the nucleic acid markers are selected from the group of markers shown in Tables A-G.

In another embodiment, the invention relates to a method of using a set of nucleic acid markers in marker-based selection for introgressing a set of alleles which are associated to a corresponding set of QTLs into maize germplasm lacking said set of alleles, wherein said alleles contribute to the phenotypic trait of grain yield, and the set of nucleic acid markers is selected from the group of markers given in SEQ ID NO: 9, 10, 13, 14, 17-20, 25-30, 35, 36, 41, 42, 47-52, 59-66, 69, 70 and 73-78 shown in Table A, which markers are represented by a polynucleotide fragment that (i) is amplified in a PCR reaction involving a pair of primers consisting of a forward and a backward primer with a nucleotide sequence as shown in Table A and (ii) has a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In another embodiment, the invention relates to a method of using a set of nucleic acid markers in marker-based selection for introgressing a set of alleles which are associated to a corresponding set of QTLs into maize germplasm lacking said set of alleles, wherein said alleles contribute to the phenotypic trait of grain moisture at harvest, and the set of nucleic acid markers is selected from the group of markers given in SEQ ID NO: 3-6, 9, 10, 13, 14, 21-24, 29-34, 37-40, 43, 44, 53, 54, 57, 58 and 65-72 shown in Table B, which markers are represented by a polynucleotide fragment that (i) is amplified in a PCR reaction involving a pair of primers consisting of a forward and a backward primer with a nucleotide sequence as shown in Table B and (ii) has a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In another embodiment, the invention relates to a method of using a set of nucleic acid markers in marker-based selection for introgressing a set of alleles which are associated to a corresponding set of QTLs into maize germplasm lacking said set of alleles, wherein said alleles contribute to the phenotypic trait of early and late root lodging, stalk lodging, and the defined set of nucleic acid markers is selected from the group of markers given in SEQ ID NO: 3, 4, 27, 28, 45-48, 59 and 60 shown in Table C, which markers are represented by a polynucleotide fragment that (i) is amplified in a PCR reaction involving a pair of primers consisting of a forward and a backward primer with a nucleotide sequence as shown in Table C and (ii) has a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In another embodiment, the invention relates to a method of using a set of nucleic acid markers in marker-based selection for introgressing a set of alleles which are associated to a corresponding set of QTLs into maize germplasm lacking said set of alleles, wherein said alleles contribute to the phenotypic trait of tassel architecture, and the defined set of nucleic acid markers is selected from the group of markers as given in SEQ ID NO: 7, 8, 11, 12, 31, 32, 39, 40, 55, 56, 81 and 82 shown in Table E, which markers are represented by a polynucleotide fragment that (i) is amplified in a PCR reaction involving a pair of primers consisting of a forward and a backward primer with a nucleotide sequence as shown in Table E and (ii) has a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In another embodiment, the invention relates to a method of using a set of nucleic acid markers in marker-based selection for introgressing a set of alleles which are associated to a corresponding set of QTLs into maize germplasm lacking said set of alleles, wherein said alleles contribute to the phenotypic trait of fungal resistance or incidence selected from the group consisting of sulcotrione resistance, fusarium ear rot incidence and common smut incidence, and the defined set of nucleic acid markers is selected from the group of markers given in SEQ ID NO: 11 and 12 shown in Table D, given in SEQ ID NO: 7, 8, 43, 44, 81 and 82 shown in Table F, and given in SEQ ID NO: 1, 2, 15, 16, 79 and 80 shown in Table G, respectively, which markers are represented by a polynucleotide fragment that (i) is amplified in a PCR reaction involving a pair of primers consisting of a forward and a backward primer with a nucleotide sequence as shown in Table D, F and G, respectively, and (ii) has a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In particular, the invention relates to a method of using one of the sets of nucleic acid markers defined herein before, particularly a set of markers which can be chosen from Tables A-G and compiled such that they are capable of detecting any one of the different sub-groups of alleles identified herein before in marker-based selection for introgressing said sub-set of alleles which are associated to a corresponding set of QTLs into maize germplasm lacking said sub-set of alleles.

In another embodiment of the invention, the maize plant according to the invention can be used as a breeding partner in a breeding program for developing new plant lines with favorable properties. One or more of the other breeding partners may be obtained from an established breeding population produced and/or used as parents in a breeding program; e.g., a commercial breeding program. The members of the established breeding population are typically well-characterized genetically and/or phenotypically. For example, several phenotypic traits of interest might have been evaluated, e.g., under different environmental conditions, at multiple locations, and/or at different times. Alternatively or in addition, one or more genetic loci associated with expression of the phenotypic traits might have been identified and one or more of the members of the breeding population might have been genotyped with respect to the one or more genetic loci as well as with respect to one or more genetic markers that are associated with the one or more genetic loci.

In one embodiment, the invention relates to a method of identifying a maize plant according to the invention and as described herein before, which method comprises the following steps:
i) obtaining plant material from a plant or a plant population to be tested and extracting DNA from said material;
ii) analyzing the DNA sample obtained in step i) to determine the allelic variants present at least 1, particularly at al least 5, more particularly at least 15, even more particularly at least 20, but especially at least 25 and up to 30-40 marker loci genetically linked to a corresponding QTL contributing to a phenotypic trait selected from the group of grain yield, grain moisture at harvest, early and late root lodging, stalk lodging, common smut incidence, fusarium ear rot incidence, sulcotrione resistance, and tassel architecture, by
   a) identifying the at least one marker locus in a PCR reaction using a pair of PCR oligonucleotide primers consisting of a forward primer and a reverse primer exhibiting a nucleotide sequence as given in SEQ ID NO: 1-82 shown in Table A-G, and
   b) identifying the marker allele by determining the molecular weight of the PCR amplification product obtained in step 1.
iii) identifying a plant or plants with the desired profile using the data of the marker analysis.

In another embodiment of the invention, DNA samples from maize plants of different genetic backgrounds other than inbred lines M3047/1 (NCIMB 41459) and M3047/2 (NCIMB 41460), are obtained and tested for the presence or absence of amplified DNA obtained in PCR amplification using primer pairs as indicated in Tables A-G, exhibiting a nucleotide sequence as given in SEQ ID NO: 1-82. Using breeding techniques known to those persons skilled in the art, plants of different maize genetic backgrounds other than from inbred lines M3047/1 (NCIMB 41459) and M3047/2 (NCIMB 41460), are the source for a set of alleles at a corresponding set of QTLs each of which contribute to a phenotypic trait of economic importance as disclosed and described herein before, wherein
a) each QTL is genetically linked to at least one marker locus, which can be identified by a pair of PCR oligonucleotide primers consisting of a forward primer and a reverse primer exhibiting a nucleotide sequence as given in SEQ ID NO: 1 - 82 shown in Tables A-G; and
b) each allele at the corresponding QTL is defined by at least one marker allele at said at least one marker locus linked to the QTL, which marker allele is characterized by the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair; and wherein said set of QTLs comprises at least 10, particularly at least 15, more particularly at least 20, even more particularly at least 25, but especially at least 30 and up to 37 different QTLs.

In a specific embodiment of the invention, the set of alleles obtained from maize plants of different genetic backgrounds can be introgressed into parental material according to marker assisted breeding techniques known to those skilled in the art. By way of example only, marker-assisted backcrossing (MABC) uses DNA markers to enable breeders to identify source material progeny that contain the desired recombinant chromosomes and donor-parent genome (Fehr 1987). Marker-assisted backcross protocols have been described by Ragot et al. (1995).

Various other methods of using markers for selecting QTLs associated with desirable traits are known to those persons skilled in the art. For example, methods of "forward breeding" with DNA markers have also been proposed and implemented by maize breeding programs. The key advantages of present-day recurrent selection methods are the availability of genetic data for all progeny at each generation of selection, the integration of genotypic and phenotypic data and the rapid cycling of generations of selection and information-directed matings at off-season nurseries.

Two distinct forms of forward breeding with MAS have been described, single large-scale marker-assisted selection (SLS-MAS) (Ribaut and Betrán 1999) and marker-assisted recurrent selection (MARS) (Edwards and Johnson 1994; Lee 1995; Stam 1995, van Berloo and Stam 1998).

Marker assisted recurrent selection (MARS) targets all traits of importance in a breeding program and for which genetic information can be obtained. Genetic information is usually obtained from QTL analyses performed on experimental populations and comes in the form of maps of QTL's with their corresponding effects. The assumption, here, is that the goal is to obtain individuals with as many accumulated favorable alleles as possible (Gallais et al. 1997; Gimelfarb and Lande 1994; Lande and Thompson 1990; Moreau et al. 1998; Xie and Xu 1998). This breeding scheme could involve several successive generations of crossing individuals (Peleman and Van Der Voort 2003; Stam 1995) and would therefore constitute what is referred to as marker-assisted recurrent selection (MARS) or genotype construction. This idea can be extended to situations where favorable alleles come from more than two parents (Peleman and Van Der Voort 2003; Stam 1995).

According to the invention, marker-based and phenotypic selection can be mobilized in many different ways, with respect to each other, in marker-assisted-based breeding schemes. Marker assisted breeding and/or phenotypic selection can be used either simultaneously or sequentially to select from maize plants of diverse genetic backgrounds, not inbred lines M3047/1 (NCIMB 41459) and M3047/2 (NCIMB 41460), one or more alleles from a set of alleles at a corresponding set of QTLs each of which contribute to a phenotypic trait of economic importance, wherein
a) each QTL is genetically linked to at least one marker locus, which can be identified by a pair of PCR oligonucleotide primers consisting of a forward primer and a reverse primer exhibiting a nucleotide sequence as given in SEQ ID NO: 1 - 82 shown in Tables A-G; and
b) each allele at the corresponding QTL is defined by at least one marker allele at said at least one marker locus linked to the QTL, which marker allele is characterized by the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair; and wherein said
set of QTLs comprises at least 10, particularly at least 15, more particularly at least 20, even more particularly at least 25, but especially at least 30 and up to 37 different QTLs.

One further method, that can be used for producing a plant according to the invention and as described herein before, is disclosed in co-pending EP application 07290060.8 filed January 17, 2007, the disclosure of which is incorporated herein by reference in its entirety.

Plants according to the invention and disclosed herein before containing a nuclear genome comprising a set of alleles at a corresponding set of QTLs each of which contribute to a phenotypic trait of economic importance selected from the group of grain yield, grain moisture at harvest, early and late root lodging, stalk lodging, common smut incidence, fusarium ear rot incidence, sulcotrione resistance, and tassel architecture, can be obtained by a method comprising the steps of
i) crossing two or more parent plants which have a genetic background capable of contributing to the development of a plant according to the invention and as described herein before, particularly crossing two parent plants which have a genetic background as represented by maize inbred lines M3047/1 (NCIMB 41459) and M3047/2 (NCIMB 41460), or an ancestor or progenitor plant thereof,
ii) screening for a plant which has in its genome a set of alleles at a corresponding set of QTLs, with each QTL being genetically-linked to at least one marker locus, wherein said set of QTLs comprises at least 10, particularly at least 15, more particularly at least 20, even more particularly at least 25, but especially at least 30 and up to 37 different QTLs, wherein each allele at the corresponding QTL is defined by at least one marker allele at said at least one marker locus linked to the QTL by
   1. identifying the at least one marker locus in a PCR reaction using a pair of PCR oligonucleotide primers consisting of a forward primer and a reverse primer exhibiting a nucleotide sequence as given in SEQ ID NO: 1-82 shown in Table A-G, and
   2. identifying the marker allele by determining the molecular weight of the PCR amplification product obtained in step 1.
iii) selecting a plant with the desired profile.

In particular, the invention relates to a method wherein at least one of the parental plants has a genome comprising a sub-set of alleles at a corresponding sub-set of QTLs genetically-linked to marker loci which can be identified in a PCR reaction using a pair of PCR oligonucleotide primers consisting of a forward primer and a reverse primer exhibiting a nucleotide sequence as given in SEQ ID NO: 1-82 shown in Table A-G, wherein said sub-set of QTLs comprises at least two QTLs, particularly at least 5, more particularly at least 10, even more particularly at least 15, but especially 20 and up to 30-37 QTLs contributing to a phenotypic trait selected from the group of grain yield, grain moisture at harvest, early and late root lodging, stalk lodging, common smut incidence, fusarium ear rot incidence, sulcotrione resistance, and tassel architecture.

Plants according to the invention may be obtained by crossing two or more parental genotypes, each of which may have a sub-set of alleles at a corresponding sub-set of QTLs, which sub-set of alleles is lacking in the other parental genotype or which complements the other genotype to obtain a plant according to the invention and as described herein before. If the two original parental genotypes do not provide the entire set of alleles, other sources can be included in the breeding population.

In a specific embodiment of the invention, the parental genotypes are from the hard flint heterotic group, but particularly consist of maize inbred lines having the invention relevant properties of inbred lines M3047/1 and M3047/2, respectively, particularly a mutually complementary set of alleles according to the invention. Seed samples of inbred lines M3047/1 and M3047/2 have been deposited with NCIMB under Accession number NCIMB 41459 and NCIMB 41460.

These parental genotypes may be crossed with one another to produce progeny seed. The parental genotypes may be inbred lines developed by selfing selected heterozygous plants from fields with uncontrolled or open pollination and employing recurrent selection procedures. Superior plants are selfed and selected in successive generations. In the succeeding generations the heterozygous condition gives way to homogeneous lines as a result of self-pollination and selection. With successive generations of inbreeding, the plant becomes more and more homozygous and uniform within the progeny plants. Typically, five to seven or more generations (F1 to F2; F3 to F4; F4 to F5) of selfing and pedigree selection may be practiced to obtain inbred lines that are uniform in plant and seed characteristics and that will remain uniform under continued self-fertilization.

During inbreeding, many undesirable recessive alleles at heterozygous loci will be replaced by dominant alleles and the recessive alleles eliminated from the progeny. Moreover, through marker-based selection the number of favorable alleles within the defined set of alleles according to the present invention can be maximized in that the more unfavourable alleles are identified and successively replaced by the more favorable alleles finally resulting in a plant containing the most preferred allele at each of the pre-determined loci within the plant genome.

QTLs are characterized by their position on the genetic map, and their additive and dominance effects. Positions are defined as a genetic distances between the most likely position of the QTLs (usually the position of the peak LOD score value) and flanking marker loci (in centimorgans). Additive and dominance effects are defined as deviations from the mean and are expressed in the same unit as the trait they refer to. Additive values define which of the parental lines carries the favorable allele at the QTL.

The origin (type) of a favorable allele can be determined at each QTL by the sign of the effect of the QTL (positive or negative) and the desirability of the trait. This allows to identify favorable alleles at each linked marker. This information can then be used to select individuals during the marker-based selection process in order to maximize the number of favorable alleles present in one individual.

For example, in case of a bi-parental cross of inbred lines, particularly of inbred lines having a mutually complementary set of alleles according to the invention such as, for example, inbred lines M3047/2 (NCIMB 41460) and M3047/1 (NCIMB 41459), additive values represent the effect an allele of one of the parental lines, which is the reference line, for example the M3047/2 (NCIMB 41460) allele, whether positive or negative. For a trait such as grain yield where the desired effect is a higher value of the trait, a positive additive value means that the reference line, for example line M3047/2 (NCIMB 41460), carries the favorable allele while a negative additive value means that the other parental line, for example M3047/1 (NCIMB 41459), carries the favorable allele. This allows to identify favorable alleles at each linked marker. These are presented in Tables A-G.

Selection in the early phases of inbred development is based largely on phenotypic characteristics that can be determined visually and are related to key performance indices such as, for example, plant vigor, lodging resistance, seed yield and quality, insect and fungal incidences, which are relevant for the susceptibility of the plant to be utilized in commercial hybrid production.

In one embodiment of the invention, grain yield, grain moisture at harvest, early root lodging, stalk lodging, common smut incidence, sulcotrione resistance, fusarium ear rot incidence and tassel architecture are recorded in phenotypic evaluation.

In the more advanced generations, particularly in the F3 to F6, more particularly the F4 generation, marker-based selection is applied followed by a phenotypic selection to identify those individuals where all of the invention relevant loci described herein before have homozygous favorable genotypes.

There are several types of molecular markers that may be used in marker-based selection including restriction fragment length polymorphism (RFLP), random amplification of polymorphic DNA (RAPD), amplified restriction fragment length polymorphism (AFLP), single sequence repeats (SSR) and single nucleotide polymorphisms SNPs.

RFLP involves the use of restriction enzymes to cut chromosomal DNA at specific short restriction sites, polymorphisms result from duplications or deletions between the sites or mutations at the restriction sites.

RAPD utilizes low stringency polymerase chain reaction (PCR) amplification with single primers of arbitrary sequence to generate strain-specific arrays of anonymous DNA fragments. The method requires only tiny DNA samples and analyses a large number of polymorphic loci.

AFLP requires digestion of cellular DNA with a restriction enzyme before using PCR and selective nucleotides in the primers to amplify specific fragments. With this method up to 100 polymorphic loci can be measured and only relatively small DNA sample are required for each test.

SSR analysis is based on DNA micro-satellites (short-repeat) sequences that are widely dispersed throughout the genome of eukaryotes, which are selectively amplified to detect variations in simple sequence repeats. Only tiny DNA samples are required for an SSR analysis. SNPs use PCR extension assays that efficiently pick up point mutations. The procedure requires little DNA per sample. One or two of the above methods may be used in a typical marker-based selection breeding programme.

The most preferred method of achieving such amplification of nucleotide fragments that span a polymorphic region of the plant genome employs the polymerase chain reaction ("PCR") (Mullis et al., Cold Spring Harbor Symp. Quant. Biol. 51:263 273 (1986);), using primer pairs involving a backward primer and a forward primer that are capable of hybridizing to the proximal sequences that define a polymorphism in its double-stranded form.

Alternative methods may be employed to amplify such fragments, such as the "Ligase Chain Reaction" ("LCR") (Barany, Proc. Natl. Acad. Sci.(U.S.A.) 88:189 193 (1991)), which uses two pairs of oligonucleotide probes to exponentially amplify a specific target. The sequences of each pair of oligonucleotides are selected to permit the pair to hybridize to abutting sequences of the same strand of the target. Such hybridization forms a substrate for a template-dependent ligase. As with PCR, the resulting products thus serve as a template in subsequent cycles and an exponential amplification of the desired sequence is obtained.

LCR can be performed with oligonucleotides having the proximal and distal sequences of the same strand of a polymorphic site. In one embodiment, either oligonucleotide will be designed to include the actual polymorphic site of the polymorphism. In such an embodiment, the reaction conditions are selected such that the oligonucleotides can be ligated together only if the target molecule either contains or lacks the specific nucleotide that is complementary to the polymorphic site present on the oligonucleotide. Alternatively, the oligonucleotides may be selected such that they do not include the polymorphic site (see, Segev, PCT Application WO 90/01069).

A further method that may alternatively be employed is the "Oligonucleotide Ligation Assay" ("OLA") (Landegren et al., Science 241:1077 1080 (1988)). The OLA protocol uses two oligonucleotides that are designed to be capable of hybridizing to abutting sequences of a single strand of a target. OLA, like LCR, is particularly suited for the detection of point mutations. Unlike LCR, however, OLA results in "linear" rather than exponential amplification of the target sequence.

Nickerson et al. have described a nucleic acid detection assay that combines attributes of PCR and OLA (Nickerson et al., Proc. Natl. Acad. Sci.(U.S.A.) 87:8923 8927 (1990)). In this method, PCR is used to achieve the exponential amplification of target DNA, which is then detected using OLA. In addition to requiring multiple, and separate, processing steps, one problem associated with such combinations is that they inherit all of the problems associated with PCR and OLA.

Schemes based on ligation of two (or more) oligonucleotides in the presence of a nucleic acid having the sequence of the resulting "di-oligonucleotide," thereby amplifying the di-oligonucleotide, are also known (Wu et al., Genomics 4:560 569 (1989)), and may be readily adapted to the purposes of the present invention.

In one embodiment, a molecular marker is a DNA fragment amplified by PCR, e.g. a SSR marker or a RAPDS marker. In one embodiment, the presence or absence of an amplified DNA fragment is indicative of the presence or absence of the trait itself or of a particular allele of the trait. In one embodiment, a difference in the length of an amplified DNA fragment is indicative of the presence of a particular allele of a trait, and thus enables to distinguish between different alleles of a trait.

In a specific embodiment of the invention simple sequence repeat (SSR) markers are used to identify invention-relevant alleles in the parent plants and/or the ancestors thereof, as well as in the progeny plants resulting from a cross of said parent plants. Simple sequence repeats are short, repeated DNA sequences and present in the genomes of all eukaryotes and consists of several to over a hundred repeats of a 1-4 nucleotide motifs. Since the number of SSRs present at a particular location in the genome often differs among plants, SSRs can be analyzed to determine the absence or presence of specific alleles.

In one aspect, the invention relates to a marker or a set of two or more markers and up to 41 markers comprising a pair of PCR oligonucleotide primers consisting of a forward primer and a reverse primer exhibiting a nucleotide sequence as given in SEQ ID NO: 1-82 shown in Tables A-G, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

In a first step, DNA samples are obtained from suitable plant material such as leaf tissue by extracting DNA using known techniques. Primers that flank a region containing SSRs within the invention-relevant QTLs disclosed herein before are then used to amplify the DNA sample using the polymerase chain reaction (PCR) method well-known to those skilled in the art.
Basically, the method of PCR amplification involves use of a pair of primers comprising two short oligonucleotide primer sequences flanking the DNA segment to be amplified. Repeated cycles of heating and denaturation of the DNA are followed by annealing of the primers to their complementary sequences at low temperatures, and extension of the annealed primers with DNA polymerase. The primers hybridize to opposite strands of the DNA target sequences. Hybridization refers to annealing of complementary DNA strands, where complementary refers to the sequence of the nucleotides such that the nucleotides of one strand can bond with the nucleotides on the opposite strand to form double stranded structures. The primers are oriented so that DNA synthesis by the polymerase proceeds bidirectionally across the nucleotide sequence between the primers. This procedure effectively doubles the amount of that DNA segment in one cycle. Because the PCR products are complementary to, and capable of binding to, the primers, each successive cycle doubles the amount of DNA synthesized in the previous cycle. The result of this procedure is exponential accumulation of a specific target fragment, that is approximately 2<n>, where n is the number of cycles.

Through PCR amplification millions of copies of the DNA segment flanked by the primers are made. Differences in the number of repeated sequences between the flanking primers in different alleles are reflected in length variations of the amplified DNA fragments. These variations can be detected by electrophoretically separating the amplified DNA fragments on gels. By analyzing the gel it can be determined whether the plant contains the desired allele in a homozygous or heterozygous state or whether the desired allele is absent from the plant genome.

Marker analysis can be done early in plant development using DNA samples extracted from leaf tissue of very young plants. This allows to identify plants with a desirable genetic make-up early in the breeding cycle and to discard plants that do not contain the desired, invention-relevant alleles prior to pollination thus reducing the size of the breeding population.

Further, by using molecular markers, a distinction can be made between homozygous recessive plants that carry two copies of the desired, invention-relevant allele and heterozygous plants that carry only one copy.

In one embodiment of the invention, the marker loci can be identified by a pair of PCR oligonucleotide primers consisting of a forward primer and a reverse primer exhibiting a nucleotide sequences as given in SEQ ID NO: 1-82 shown in Tables A-G or the nucleic acid complements the sequences given in SEQ ID NO: 1-82, or fragments thereof, including oligonucleotide primers consisting of a forward primer and a reverse primer exhibiting a nucleotide sequences that share between 90% and 99%, particularly between 95% and 98% sequence identity with the nucleotide sequences given in SEQ ID NO: 1-82.

Further can be used within the scope of the invention oligonucleotide primers consisting of a forward primer and a reverse primer exhibiting a nucleotide sequences that hybridize to the nucleotide sequences of the forward and reverse primer sequences given in SEQ ID NO: 1-82 shown in Tables A-G under high stringency conditions.

In particular, the hybridization reaction is carried out under high stringency conditions at which 5xSSPE, 1% SDS, 1xDenhardts solution is used as a solution and/or hybridization temperatures are between 35°C and 70°C, and up to 72°C, preferably 65°C. After hybridization, washing is particularly carried out first with 2xSSC, 1% SDS and subsequently with 0.2xSSC at temperatures between 35°C and 70°C, and up to 72°C, particularly at 65°C (regarding the definition of SSPE, SSC and Denhardts solution see Sambrook et al. loc. cit.).

Alternative markers can be developed and used to identify and select plants with an allele or a set of alleles of a quantitative trait locus according to the present invention and as disclosed herein before.

For example, the nucleotide sequence of the amplification product obtained in PCR amplification using the primer pairs as indicated in Tables A-G, exhibiting a nucleotide sequence as given in SEQ ID NO: 1-82, can be obtained by those skilled in the art and new primers or primer pairs designed based on the newly determined nucleotide sequence of the PCR amplification product.

To determine the utility of the inbred line and its potential to genetically contribute to the hybrid progeny a test-cross is made with another inbred line, particularly an inbred line from a different heterotic group, and the resulting progeny phenotypically evaluated. Traits that may be recorded commonly involve traits that are related to plant vigor and productiveness including grain yield, grain moisture at harvest, early and late root lodging, stalk lodging, common smut incidence, fusarium ear rot incidence, sulcotrione resistance, and tassel architecture, but particularly grain yield, grain moisture at harvest, late root lodging, and stalk lodging.

In a specific embodiment of the invention, a plant according to the invention and as disclosed herein before is produced through a bi-parental cross of inbred lines, particularly of inbred lines having the invention relevant alleles of lines M3047/2 (NCIMB 41460) and M3047/1 (NCIMB 41459). F₁ kernels are harvested and replanted. The resulting F₁ plants are grown to maturity and self-fertilized to produce F₂ seed.

A defined and limited number of F₂ kernels, particularly between 200 and 1000, more particularly between 300 and 600, but especially 500, are replanted. The resulting F₂ plants are again grown to maturity and self-fertilized to produce F₃ seed.

After that a commonly-used generation advancement procedure may be applied such as that known as single kernel descent (SKD). In this procedure, only one F₃ kernel is harvested on each F₂ plant. The F₃ kernels harvested are planted, and the resulting F₃ plants self-fertilized to produce F₄ seed. All F₄ kernels produced on each F₃ plant are harvested, keeping all F₄ kernels harvested separated by F₃ plant of origin, and thereby constituting F₄ families.

Plants from each F₄ family are then grown. A part of the resulting plants is used later to collect leaf tissue used for DNA extraction and genotyping. Another part of said plants is crossed to a tester plant, particularly a maize inbred line from a different heterotic group than that of the two parental inbred lines, particularly an inbred line from the lodent heterotic group such as, for example, FSII434,. F₄ plants are de-tasseled and thereby used as females, while the tester is used as the male to pollinate all F₄ plants. Testcross seed was harvested, maintaining the family structure.

Testcross seed from the F₄ families are planted and evaluated in the field preferably under different growing and climatic conditions. Several other hybrids, used as checks, may also be planted in the same trials.

Traits recorded include grain yield, grain moisture at harvest, early and late root lodging, stalk lodging, common smut incidence, fusarium ear rot incidence, sulcotrione resistance, and tassel architecture. Grain yield, grain moisture at harvest, late root lodging, and stalk lodging were recorded on testcross plots, particularly early root lodging, stalk lodging, common smut incidence, sulcotrione resistance, and tassel architecture. Fusarium ear rot incidence was recorded both on testcross plots and F₄ plots.

A subset of QTLs is selected from all QTLs identified. The position of these QTLs relative to neighboring markers, along with their effects and favorable alleles are represented in Tables 1 to 8. These QTLs are the selection targets used to develop new lines.

For genotyping and QTL mapping DNA is extracted from suitable plant material such as, for example, leaf tissue. In particular, bulks of leaves of a plurality of plants are collected for each F₄ family. DNA samples are genotyped using a plurality of polymorphic SSR's covering the entire maize genome, particularly between 80 and 250, particularly between 90 and 200, more particularly between 100 and 150, but especially 112 SSRs.
A molecular marker map can be constructed using the commonly used software such as, for example, Mapmaker and Joinmap. This molecular marker map had a total length of 2,187 centimorgans (cM), with a marker density of one marker every 19.5 cM.

Joint-analysis of genotypic and phenotypic data can be performed using standard software such as, for example, the software QTLCartographer and PlabQTL. One hundred and thirty QTLs are identified, for all traits. In particular, 23 QTLs are identified for grain yield, and 40 for grain moisture. QTLs are characterized by their position on the genetic map, and their additive and dominance effects. Positions are defined as a genetic distances between the most likely position of the QTLs (usually the position of the peak LOD score value) and flanking marker loci (in centimorgans). Additive and dominance effects are defined as deviations from the mean and are expressed in the same unit as the trait they refer to. Additive values define which of the two parental lines carries the favorable allele at the QTL. In a specific embodiment of the invention, additive values represent the effect of the M3047/2 (NCIMB 41460) allele, whether positive or negative. For a trait such as grain yield where the desired effect is a higher value of the trait, a positive additive value means that M3047/2 (NCIMB 41460) carries the favorable allele while a negative additive value means that M3047/1 (NCIMB 41459) carries the favorable allele.

Starting with F₄ individuals resulting from the initial cross between inbred lines exhibiting the unique QTL profile according to the invention, but particularly inbred lines M3047/1 (NCIMB 41459) and M3047/2 (NCIMB 41460), marker-based selection is applied followed by phenotypic selection. Several inbred lines may be developed for which all of the above loci have homozygous favorable genotypes. These inbred lines can the be subjected to a testcrossing procedure where the are crossed with several tester plants and tested in the field under different climatic and environmental conditions for their agronomic performance, and compared with other hybrids.

The most desirable hybrids are those which show high grain yield and low grain moisture at harvest.

Plant introductions and germplasm can be screened for the alleles at the corresponding QTLs disclosed in Tables 1 to 8 based on the nucleotide sequence of the marker at the marker locus linked to said QTL and the molecular weight of allele using one or more of techniques disclosed herein or known to those skilled in the art.

### FIGURES

### Figure 1.

Agronomic performance of marker-based-selection-derived material, compared to reference material. The figure shows grain yield (in quintals per hectare) and grain moisture at harvest of hybrids made from four marker-based-selection-derived lines, ILD01, ILD02, ILD06, and ILD07, crossed onto three testers, TSTR01, TSTR04, and TSTR06, and grown at 8 locations in France in 2006. The results shown are the averages over all 8 locations. The figure also shows performance of reference (check) hybrids. Check hybrids are represented by black diamonds. Marker-based-selection-derived hybrids are represented by white squares.
The most desirable hybrids are those which show high grain yield and low grain moisture at harvest, therefore positioned in the upper left corner of the figure. Most of the hybrids in this area of the figure are made from marker-based-selection-derived lines.

### DEPOSITS

A representative sample of seeds of maize inbred line M3047/1 has been deposited under the provisions of the Budapest treaty with NCIMB, Aberdeen, AB24 3RY, Scotland on January 15, 2007 under Accession number NCIMB 41459.

A representative sample of seeds of maize inbred line M3047/2 has been deposited under the provisions of the Budapest treaty with NCIMB, Aberdeen, AB24 3RY, Scotland on January 15, 2007 under Accession number NCIMB 41460.

### EXAMPLES

### Identification and use of QTL to derive superior inbred lines and hybrids

### Plant Material

Parental material consisted of two maize inbred lines: M3047/1 (NCIMB 41459) and M3047/2 (NCIMB 41460), both from the hard flint heterotic group.
These lines were crossed with one another to produce F₁ seed.
F₁ kernels were planted and the resulting F₁ plants were self-fertilized to produce F₂ seed.
About 500 F₂ kernels were planted. The resulting F₂ plants were self-fertilized to produce F₃ seed.
One and only one F₃ kernel was harvested on each F₂ plant, a commonly-used generation advancement procedure known as single kernel descent (SKD). The almost 500 F₃ kernels so harvested were planted, and the resulting F₃ plants self-fertilized to produce F₄ seed. All F₄ kernels produced on each F₃ plant were harvested, keeping all F₄ kernels harvested separated by F₃ plant of origin, and thereby constituting F₄ families.
About 10 kernels from each F₄ family were planted to collect leaf tissue later used for DNA extraction and genotyping.
About 25 kernels from 260 unselected F₄ families were planted in an isolated field to be crossed to a tester (a maize inbred line from a different heterotic group than that of the two parental inbred lines of the project): FSII434, from the lodent heterotic group. F₄ plants were de-tasseled and thereby used as females, while the tester was used as the male to pollinate all F₄ plants. Testcross seed was harvested, maintaining the family structure.

### Phenotypic Evaluations

Testcross seed from 260 F₄ families was planted at 6 field locations in 1998, in two-row plots. The experimental design was a lattice design with one replication. Several other hybrids, used as checks, were also planted in the same trials.
Seed from the same 260 F₄ families was also planted at two field locations in 1998, in one-row plots. Several inbred lines, used as checks, were also planted at the same location.
Traits recorded included grain yield, grain moisture at harvest, early vigor, male and female flowering dates, early and late root lodging, stalk lodging, common smut incidence, fusarium ear rot incidence, sulcotrione resistance, and tassel architecture. Grain yield, grain moisture at harvest, late root lodging, and stalk lodging were recorded on testcross plots. Early vigor, male and female flowering dates, early root lodging, stalk lodging, common smut incidence, sulcotrione resistance, and tassel architecture were recorded on F₄ plots. Fusarium ear rot incidence was recorded both on testcross plots and F₄ plots.

### Genotyping and QTL Mapping

DNA was extracted from bulks of leaves of about 10 F₄ plants for each F₄ family. DNA samples were genotyped using 112 polymorphic SSR's covering the entire maize genome. Several hundred SSR's had been previously run on the two parents of this segregating population, M3047/1 (NCIMB 41459) and M3047/2 (NCIMB 41460), in order to identify the polymorphic ones. The molecular marker genotypes obtained from analyses of F₄ DNA bulks represented the genotypes of the F₃ plants from which F₄ families had been derived.
A molecular marker map was constructed using the commonly used software Mapmaker and Joinmap. This molecular marker map had a total length of 2,187 centimorgans (cM), with a marker density of one marker every 19.5 cM.
Joint-analysis of genotypic and phenotypic data was performed using the software QTLCartographer and PlabQTL. One hundred and thirty QTLs were identified, for all traits. In particular, 23 QTLs were identified for grain yield, and 40 for grain moisture. QTLs are characterized by their position on the genetic map, and their additive and dominance effects. Positions are defined as a genetic distances between the most likely position of the QTLs (usually the position of the peak LOD score value) and flanking marker loci (in centimorgans). Additive and dominance effects are defined as deviations from the mean and are expressed in the same unit as the trait they refer to. Additive values define which of the two parental lines carries the favorable allele at the QTL. In this case additive values represent the effect of the M3047/2 (NCIMB 41460) allele, whether positive or negative. For a trait such as grain yield where the desired effect is a higher value of the trait, a positive additive value means that M3047/2 (NCIMB 41460) carries the favorable allele while a negative additive value means that M3047/1 (NCIMB 41459) carries the favorable allele.

### Marker-Based Selection

A subset of QTLs was selected from all QTLs identified. The position of these QTLs relative to neighboring markers, along with their effects and favorable alleles, are represented in Tables 1 to 8. These QTLs were the selection targets used to develop new lines.

### Table 1.

QTLs for grain yield and linked markers. Each grain yield QTL is assigned an arbitrary number. The following information is given for each QTL: the chromosome on which it is located, its most significant position on that chromosome, the beginning and end of its confidence interval, its effect (additive value) as characterized by the difference between the effect of the allele from M3047/2 (NCIMB 41460) and that of the allele from M3047/1 (NCIMB 41459), and markers linked to the QTL (and therefore diagnostic of the allele present at the QTL).

For instance, the first QTL for grain yield is located on chromosome 1 with a most likely position at 115,6cM but a confidence interval ranging from 110,6cM to 120,6cM. The effect of the QTL is 1.94, which means that the allele M3047/2 (NCIMB 41460) increase grain yield by 1.94%, compared to the allele from M3047/1 (NCIMB 41459). In this case the favorable allele comes from M3047/2 (NCIMB 41460).

| Grain Yield QTL # | Chromosome | Map Position (cM) | QTL Begin (cM) | QTL End (cM) | Effect (Add Value) | Linked Markers | |
|---|---|---|---|---|---|---|---|
| 1 | 1 | 115,6 | 110,6 | 120,6 | 1,94 | M59/60-2 | M77/78-2 |
| 2 | 1 | 127,9 | 123,9 | 134,9 | 7,63 | M77/78-2 | M27/28-2 |
| 3 | 1 | 156,8 | 147,8 | 165,7 | 9,32 | M47/48-2 | M75/76-2 |
| 4 | 1 | 260,8 | 258,8 | 265,8 | 1,68 | M65/66-2 | M9/10-2 |
| 5 | 2 | 52,7 | 50,7 | 61,7 | -6,05 | M69/70-1 | M13/14-1 |
| 6 | 2 | 165,2 | 160,2 | 169,7 | -1,94 | M73/74-1 | M25/26-1 |
| 7 | 4 | 165,3 | 160,3 | 170,3 | -2,51 | M35/36-1 | M63/64-1 |
| 8 | 4 | 185,3 | 180,3 | 200,3 | -2,11 | M35/36-1 | M63/64-1 |
| 9 | 4 | 207,3 | 202,3 | 212,3 | -2,83 | M35/36-1 | M63/64-1 |
| 10 | 5 | 42,9 | 37,9 | 47,9 | -1,91 | M41/42-1 | M49/50-1 |
| 11 | 5 | 54,4 | 49,4 | 61,9 | -2,03 | M40/50-1 | M61/62-1 |
| 12 | 5 | 218,8 | 209,8 | 220,4 | -2,36 | M17/18-1 | M51/52-1 |
| 13 | 5 | 230,4 | 225,4 | 234,0 | -1,66 | M51/52-1 | M19/20-1 |
| 14 | 7 | 137,3 | 132,3 | 141,9 | -9,07 | M29/30-1 | |

### Table 2.

QTLs for grain moisture at harvest and linked markers. Each grain yield QTL is assigned an arbitrary number. The following information is given for each QTL: the chromosome on which it is located, its most significant position on that chromosome, the beginning and end of ts confidence interval, its effect (additive value), and markers linked to the QTL (and therefore diagnostic of the allele present at the QTL).

| Grain Moisture QTL # | Chromosome | Map Position (cM) | QTL Begin (cM) | QTL End (cM) | Effect (Add Value) | Linked Markers | |
|---|---|---|---|---|---|---|---|
| 1 | 1 | 51,9 | 46,9 | 62,6 | -0,28 | M23/24-2 | M3/4-2 |
| 2 | 1 | 257,5 | 252,5 | 271,8 | -0,28 | M65/66-2 | M9/10-2 |
| 3 | 2 | 52,7 | 50,7 | 61,7 | 0,31 | M69/70-1 | M13/14-1 |
| 4 | 2 | 225,5 | 221,5 | 230,5 | 0,18 | M71/72-1 | M53/54-1 |
| 5 | 2 | 252,5 | 237,5 | 254,5 | 0,25 | M53/54-1 | M57/58-1 |
| 6 | 3 | 185,7 | 180,7 | 190,7 | -0,28 | M43/44-2 | |
| 7 | 4 | 99,4 | 94,4 | 104,4 | 0,19 | M5/6-1 | M37/38-1 |
| 8 | 5 | 180,7 | 175,7 | 185,7 | 0,26 | M21/22-1 | M33/34-1 |
| 9 | 7 | 81,0 | 64,0 | 92,0 | 0,34 | M31/32-1 | M39/40-1 |
| 10 | 7 | 141,3 | 136,3 | 141,9 | 0,21 | M29/30-1 | |
| 11 | 8 | 62,8 | 53,8 | 67,8 | -0,28 | M67/68-2 | |

### Table 3.

QTLs for root and stalk lodging and linked markers. Each grain yield QTL is assigned an arbitrary number. The following information is given for each QTL: the chromosome on which it is located, its most significant position on that chromosome, the beginning and end of ts confidence interval, its effect (additive value), and markers linked to the QTL (and therefore diagnostic of the allele present at the QTL).

| Root/Stalk Lodging QTL # | Chromosome | Map Position (cM) | QTL Begin (cM) | QTL End (cM) | Effect (Add Value) | Linked Markers | |
|---|---|---|---|---|---|---|---|
| 1 | 1 | 73,7 | 68,7 | 77,6 | -6,70 | M3/4-2 | M59/60-2 |
| 2 | 1 | 142,8 | 137,8 | 147,8 | -2,53 | M27/28-2 | M47/48-2 |
| 3 | 1 | 224,6 | 219,6 | 229,6 | 2,74 | M45/46-1 | |

### Table 4.

QTLs for common smut incidence and linked markers. Each grain yield QTL is assigned an arbitrary number. The following information is given for each QTL: the chromosome on which it is located, its most significant position on that chromosome, the beginning and end of ts confidence interval, its effect (additive value), and markers linked to the QTL (and therefore diagnostic of the allele present at the QTL).

| Common Smut Incidence QTL # | Chromosome | Map Position (cM) | QTL Begin (cM) | QTL End (cM) | Effect (Add Value) | Linked Markers |
|---|---|---|---|---|---|---|
| 1 | 3 | 79,0 | 74,0 | 84,0 | 1,00 | M11/12-1 |

### Table 5.

QTLs for tassel architecture and linked markers. Each grain yield QTL is assigned an arbitrary number. The following information is given for each QTL: the chromosome on which it is located, its most significant position on that chromosome, the beginin and end of ts confidence interval, its effect (additive value), and markers linked to the QTL (and therefore diagnostic of the allele present at the QTL).

| Tassel Architecture QTL # | Chromosome | Map Position (cM) | QTL Begin (cM) | QTL End (cM) | Effect (Add Value) | Linked Markers | |
|---|---|---|---|---|---|---|---|
| 1 | 3 | 78,3 | 73,3 | 83,3 | -0,42 | M11/12-1 | |
| 2 | 6 | 199,1 | 195,1 | 204,1 | -0,47 | M55/56-1 | |
| 3 | 7 | 85,0 | 80,0 | 90,0 | -0,41 | M31/32-1 | M39/40-1 |
| 4 | 9 | 10,0 | 5,0 | 15,0 | -0,36 | M81/82-1 | M7/8-1 |

### Table 6.

QTLs for sulcotrione resistance and linked markers. Each grain yield QTL is assigned an arbitrary number. The following information is given for each QTL: the chromosome on which it is located, its most significant position on that chromosome, the beginning and end of ts confidence interval, its effect (additive value), and markers linked to the QTL (and therefore diagnostic of the allele present at the QTL).

| Sulcotrione Resistance QTL # | Chromosome | Map Position (cM) | QTL Begin (cM) | QTL End (cM) | Effect (Add Value) | Linked Markers | |
|---|---|---|---|---|---|---|---|
| 1 | 3 | 187,7 | 182,7 | 192,7 | -0,38 | M43/44-2 | |
| 2 | 9 | 35,7 | 30,7 | 40,7 | 0,35 | M81/82-1 | M7/8-1 |

### Table 7.

QTLs for fusarium ear rot incidence and linked markers. Each grain yield QTL is assigned an arbitrary number. The following information is given for each QTL: the chromosome on which it is located, its most significant position on that chromosome, the beginning and end of ts confidence interval, its effect (additive value), and markers linked to the QTL (and therefore diagnostic of the allele present at the QTL).

| Fusarium Ear Rot Incidence QTL # | Chromosome | Map Position (cM) | QTL Begin (cM) | QTL End (cM) | Effect (Add Value) | Linked Markers | |
|---|---|---|---|---|---|---|---|
| 1 | 5 | 122,1 | 117,1 | 127,1 | 1,76 | M1/2-1 | M79/80-1 |
| 2 | 5 | 140,6 | 135,6 | 145,6 | 0,54 | M79/80-1 | M15/16-1 |

The origin (type) of favorable allele was determined at each QTL by the sign of the effect of the QTL (positive or negative) and the desirability of the trait. This allowed to identify favorable alleles at each linked marker. These are presented in Tables A-G. This information was used to select individuals during the marker-based selection process, the objective of which is to maximize the number of favorable alleles present in one individual.

### Performance of Marker-Based-Selection-Derived Material

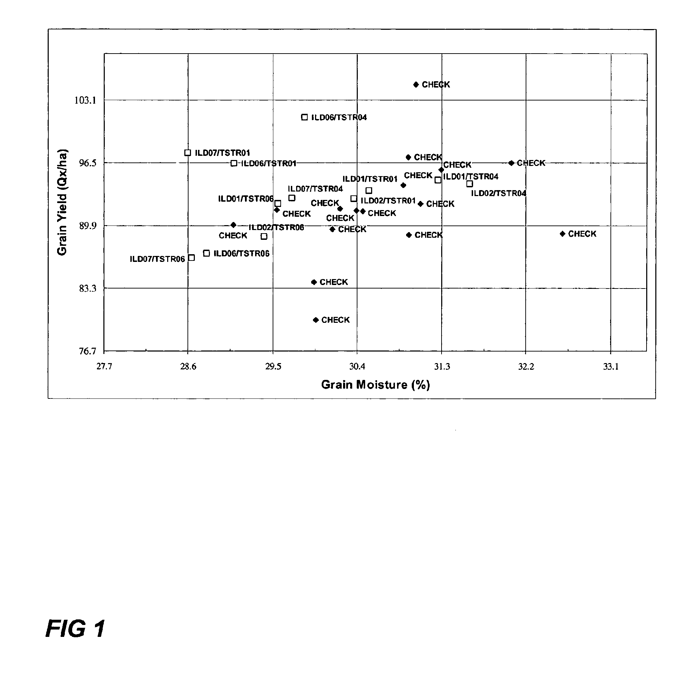

Marker-based selection followed by phenotypic selection was conducted starting with F₄ individuals from the cross between inbred lines M3047/1 (NCIMB 41459) and M3047/2 (NCIMB 41460). Several inbred lines were developed for which all of the above loci have homozygous favorable genotypes. These inbred lines were testcrossed to several testers, tested in the field for their agronomic performance, and compared with other hybrids. Results from four inbred lines, ILD01, ILD02, ILD06, and ILD07 are presented in Figure 1. Field testing was conducted at 8 locations in France in 2006. The allelic composition of these four lines at markers flanking QTLs is that presented in Table 9 above.

### Example protocol for determination of allele characteristics (size) using agarose

3µl of DNA (concentration of 2ng/µl) is distributed in 384-well plates.
3µl of "PCR mix" is also added to the wells. The composition of the "PCR mix" is as described I the following table:

| Ingredient | Concentration | Product Reference |
|---|---|---|
| PCR Buffer | 1 x | Invitrogen PCR Buffer/réf10966083 |
| MgCl2 | 1.65 mM | |
| dNTP | 62.5 µM each | |
| Taq Polymerase | 0.033 U/µl | Invitrogen platinium Taq / réf10966083 |
| Primers (Forward, Reverse) | 412 nM each | |

PCR amplification is conducted with thermocycler GeneAmp PCR System 9700 from Applied Biosystems and comprises the following steps:
- 2 minutes at 94°C
- 40 cycles of 15 seconds at 94°C followed by 45 seconds at 59°C
- 2 minutes at 72°C

PCR amplification products are separated on agarose gels using high resolution agarose at a concentration of 3% in TBE (tris-borate EDTA) 1X. Agarose is purchased from Invitrogen (Agarose 100, reference 10975). Electrophoresis is conducted at 400 volts during 1 hour.
PCR amplification products are revealed after migration using ethidium bromide and viewing under UV light.

### Example protocol for determination of allele characteristics (molecular weight) using a sequencer

5µl of DNA (concentration of 2ng/µl) is distributed in 384-well plates.
5µl of "PCR mix" is also added to the wells. The composition of the "PCR mix" is as described I the following table:

| Ingredient | Concentration | Product Reference |
|---|---|---|
| PCR Buffer | 1 x | Invitrogen PCR Buffer/réf10966083 |
| Mgcl2 | 1.65 mM | |
| dNTP | 0.2 mM each | |
| Taq Polymerase | 0.033 U/µl | Invitrogen platinium Taq / réf10966083 |
| Primers (Forward M13) | 800 nM | |
| Primers (Reverse) | 600 nM | |
| Fluorescent M13 Probe | 600 nM | |

PCR amplification is conducted with thermocycler GeneAmp PCR System 9700 from Applied Biosystems and comprises the following steps:
- 2 minutes at 94°C
- 40 cycles of 15 seconds at 94°C followed by 45 seconds at 59°C
- 2 minutes at 72°C

PCR amplification products are first denatured with formamide during 3 minutes at 96°C before being separated on a sequencer AbiPrism 3700 from Applied Biosystems. Migration in the sequencer takes place in capillaries filled with polymer POP6 (purchased from Applied Biosystems, reference 4311320) and TBE 1X. Molecular weights of the PCR amplification fragments are determined using software Genescan and Genotyper.

**Table 8: Molecular weight (in base pairs) of PCR amplification products of favorable alleles at molecular markers linked to QTLs.**

| Linked Marker | Favorable Allele | Molecular Weight (bp) |
|---|---|---|
| M1/2-1 | FSNU505 | 175 |
| M3/4-2 | FPWW513 | 70 |
| M5/6-1 | FSNU505 | 250 |
| M7/8-1 | FSNU505 | 100 |
| M9/10-2 | FPWW513 | 70 |
| M11/12-1 | FSNU505 | 100 |
| M13/14-1 | FSNU505 | 350 |
| M15/16-1 | FSNU505 | 90 |
| M17/18-1 | FSNU505 | 100 |
| M19/20-1 | FSNU505 | 100 |
| M21/22-1 | FSNU505 | 150 |
| M23/24-2 | FPWW513 | |
| M25/26-1 | FSNU505 | 125 |
| M27/28-2 | FPWW513 | 350 |
| M29/30-1 | FSNU505 | 225 |
| M31/32-1 | FSNU505 | 160 |
| M33/34-1 | FSNU505 | 225 |
| M35/36-1 | FSNU505 | 215 |
| M37/38-1 | FSNU505 | 205 |
| M39/40-1 | FSNU505 | 125 |
| M41/42-1 | FSNU505 | 225 |
| M43/44-2 | FPWW513 | 155 |
| M45/46-1 | FSNU505 | 185 |
| M47/48-2 | FPWW513 | 130 |
| M40/50-1 | FSNU505 | 120 |
| M51/52-1 | FSNU505 | 240 |
| M53/54-1 | FSNU505 | 90 |
| M55/56-1 | FSNU505 | 110 |
| M57/58-1 | FSNU505 | 125 |
| M59/60-2 | FPWW513 | 120 |
| M61/62-1 | FSNU505 | 160 |
| M63/64-1 | FSNU505 | 160 |
| M65/66-2 | FPWW513 | 200 |
| M67/68-2 | FPWW513 | 250 |
| M69/70-1 | FSNU505 | 175 |
| M73/74-1 | FSNU505 | 135 |
| M75/76-2 | FPWW513 | 170 |
| M77/78-2 | FPWW513 | 85 |
| M79/80-1 | FSNU505 | 220 |
| M81/82-1 | FSNU505 | 105 |

Molecular weights indicated here are the result of onw PCR amplification followed by migration on an agarose gel according to the above protocol.

These molecular weights are thus estimates of the exact molecular weights and variation around the values indicated here are likely to be observed if the agarose migration were to be repeated.

**Table A QTLs for Grain Yield, Favorable Alleles and linked Markers**

| Grain Yield QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 1 | M3047/2 | M59/60-2 | CCCAGCGCATGTCAACTCT | SEQ.ID.NO: 59 | CCCCGGTAATTCAGTGGATA | SEQ.ID.NO: 60 |
| 1 | 1 | M3047/2 | M77/78-2 | TTGCACCCCGTTATTATCCTACAG | SEQ.ID.NO: 77 | CCAGACTAGAGTGCCATGATCCTT | SEQ.ID.NO: 78 |
| 2 | 1 | M3047/2 | M77/78-2 | TTGCACCCCGTTATTATCCTACAG | SEQ.ID.NO: 77 | CCAGACTAGAGTGCCATGATCCTT | SEQ.ID.NO: 78 |
| 2 | 1 | M3047/2 | M27/28-2 | TTCACCGCCTCACATGAC | SEQ.ID.NO: 27 | GCAACGCTAGCTAGCTTTG | SEQ.ID.NO: 28 |
| 3 | 1 | M3047/2 | M47/48-2 | CAGAAGGGGAGGAGGGATAC | SEQ.ID.NO: 47 | ATTATGCTCAAGCACAGGGC | SEQ.ID.NO: 48 |
| 3 | 1 | M3047/2 | M75/76-2 | ATATCTTCTTCTTGTCCTCCG | SEO.ID.NO: 75 | CATCCCCTTATCCCTCC | SEO.ID.NO: 76 |
| 4 | 1 | M3047/2 | M65/66-2 | ACAGCACTGGGAACCAAAAC | SEQ.ID.NO: 65 | ATCCCCTCTTCCATCTCTGC | SEQ.ID.NO: 66 |
| 4 | 1 | M3047/2 | M9/10-2 | CCGAATTGAAATAGCTGCGAGAACCT | SEQ.ID.NO: 9 | ACAATGAACGGTGGTTATCAACACGC | SEQ.ID.NO: 10 |
| 5 | 2 | M3047/1 | M69/70-1 | TTACGGTACCAATTCGCTCC | SEQ.ID.NO: 69 | GACGACGCCATTTTCTGATT | SEQ.ID.NO: 70 |
| 5 | 2 | M3047/1 | M13/14-1 | CTGCTCTCACTGAGCTTGATGGAAAGG | SEO.ID.NO: 13 | TGCAAATCAATGGCAAGGGACCTCGTAGTT | SEQ.ID.NO: 14 |
| 6 | 2 | M3047/1 | M73/74-1 | TCGTCGTCTCCAATCATACGTG | SEQ.ID.NO: 73 | GCTACACGATACCATGGCGTTT | SEQ.ID.NO: 74 |
| 6 | 2 | M3047/1 | M25/26-1 | GGGAGTATGGTAGGGAACCC | SEQ.ID.NO: 25 | AAACCCTTGGAGCATACCCT | SEQ.ID.NO: 26 |
| 7 | 4 | M3047/1 | M35/36-1 | CGTTACCCATTCCTGCTACG | SEQ.ID.NO: 35 | CTTGCTCGTTTCCATTCCAT | SEQ.ID.NO: 36 |
| 7 | 4 | M3047/1 | M63/64-1 | ACCGGAACAGACGAGCTCTA | SEQ.ID.NO: 63 | GTCCTGCAAAGCAACCTAGC | SEQ.ID.NO: 64 |
| 8 | 4 | M3047/1 | M35/36-1 | CGTTACCCATTCCTGCTACG | SEQ.ID.NO: 35 | CTTGCTCGTTTCCATTCCAT | SEQ.ID.NO: 36 |
| 8 | 4 | M3047/1 | M63/64-1 | ACCGGAACAGACGAGCTCTA | SEQ.ID.NO: 63 | GTCCTGCAAAGCAACCTAGC | SEQ.ID.NO: 64 |
| 9 | 4 | M3047/1 | M35/36-1 | CGTTACCCATTCCTGCTACG | SEQ.ID.NO: 35 | CTTGCTCGTTTCCATTCCAT | SEQ.ID.NO: 36 |
| 9 | 4 | M3047/1 | M63/64-1 | ACCGGAACAGACGAGCTCTA | SEQ.ID.NO: 63 | GTCCTGCAAAGCAACCTAGC | SEQ.ID.NO: 64 |
| 10 | 5 | M3047/1 | M41/42-1 | TTTTCTTTCAAAAATATTCAGAAGC | SEQ.ID.NO: 41 | GCAGGATTTCATCGGTTGTT | SEQ.ID.NO: 42 |
| 10 | 5 | M3047/1 | M49/50-1 | AACCAAGGTTCTTGGAGGCT | SEQ.ID.NO: 49 | ACCATTGTATTTTCCTAGAGAATCG | SEQ.ID.NO: 50 |
| 11 | 5 | M3047/1 | M40/50-1 | AACCAAGGTTCTTGGAGGCT | SEQ.ID.NO: 49 | ACCATTGTATTTTCCTAGAGAATCG | SEO.ID.NO: 50 |
| 11 | 5 | M3047/1 | M61/62-1 | TGCTCTCACAAGATGGTGGA | SEQ.ID.NO: 61 | CCACAGGATAAAATCGGCTG | SEQ.ID.NO: 62 |
| 12 | 5 | M3047/1 | M17/18-1 | CTTCCAGCCGCAACCCTC | SEQ.ID.NO: 17 | CCAACAACGCGGACGTGA | SEQ.ID.NO: 18 |
| 12 | 5 | M3047/1 | M51/52-1 | TAATCTTGGGGGGTTTAGGG | SEQ.ID.NO: 51 | GACATGTCCCATTCCCATTC | SEQ.ID.NO: 52 |
| 13 | 5 | M3047/1 | M51/52-1 | TAATCTTGGGGGGTTTAGGG | SEQ.ID.NO: 51 | GACATGTCCCATTCCCATTC | SEQ.ID.NO: 52 |
| 13 | 5 | M3047/1 | M19/20-1 | GGTCACCCTCCCTTTGCAG | SEQ.ID.NO: 19 | ATTGCCTACACAGTTTGATTGG | SEQ.ID.NO: 20 |
| 14 | 7 | M3047/1 | M29/30-1 | TTCCAGTAAGGGAGGTGCTG | SEQ.ID.NO: 29 | TAAGCAACATATAGCCGGGC | SEQ.ID.NO: 30 |

**Table B QTLs for Grain Moisture at Harvest, Favorable Alleles and Linked Markers**

| Grain Moisture at Harvest QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 1 | M3047/2 | M23/24-2 | GATGCAATAAAGGTTGCCGT | SEO.ID.NO: 23 | ATGTGCTGTGCCTGCCTC | SEQ.ID.NO: 24 |
| 1 | 1 | M3047/2 | M3/4-2 | TGACGGACGTGGATCGCTTCAC | SEQ.ID.NO: 3 | AGCAGGCAGCAGGTCAGCAGCG | SEQ.ID.NO: 4 |
| 2 | 1 | M3047/2 | M65/66-2 | ACAGCACTGGGAACCAAAAC | SEQ.ID.NO: 65 | ATCCCCTCTTCCATCTCTGC | SEQ.ID.NO: 66 |
| 2 | 1 | M3047/2 | M9/10-2 | CCGAATTGAAATAGCTGCGAGAACCT | SEQ.ID.NO: 9 | ACAATGAACGGTGGTTATCAACACGC | SEQ.ID.NO: 10 |
| 3 | 2 | M3047/1 | M69/70-1 | TTACGGTACCAATTCGCTCC | SEQ.ID.NO: 69 | GACGACGCCATTTTCTGATT | SEQ.ID.NO: 70 |
| 3 | 2 | M3047/1 | M13/14-1 | CTGCTCTCACTGAGCTTGATGGAAAGG | SEQ.ID.NO: 13 | TGCAAATCAATGGCAAGGGACCTCGTAGTT | SEQ.ID.NO: 14 |
| 4 | 2 | M3047/1 | M71/72-1 | GAGAAGAGGTGGACAAACTCT | SEQ.ID.NO: 71 | TGGAGGTAGAAGAGAATTGTG | SEQ.ID.NO: 72 |
| 4 | 2 | M3047/1 | M53/54-1 | ACGACTTTCATGCCTCGTCT | SEQ.ID.NO: 53 | ATTTCTTTTGCCACCTCAGC | SEQ.ID.NO: 54 |
| 5 | 2 | M3047/1 | M53/54-1 | ACGACTTTCATGCCTCGTCT | SEQ.ID.NO: 53 | ATTTCTTTTGCCACCTCAGC | SEQ.ID.NO: 54 |
| 5 | 2 | M3047/1 | M57/58-1 | ACAGCTTTAGACTTAGACCACACG | SEQ.ID.NO: 57 | GCACAAGCGAAGGTTTTCTC | SEQ.ID.NO: 58 |
| 6 | 3 | M3047/2 | M43/44-2 | CTGGGCAGACAGCAACAGTA | SEQ.ID.NO: 43 | AGCCAAAGACATGATGGTCC | SEQ.ID.NO: 44 |
| 7 | 4 | M3047/1 | M5/6-1 | TAATTCCTCGCTCCCGGATTCAGC | SEQ.ID.NO: 5 | GTGCATGAGGGAGCAGCAGGTAGTG | SEQ.ID.NO: 6 |
| 7 | 4 | M3047/1 | M37/38-1 | AGCTGATCTGCACGTTGTTG | SEQ.ID.NO: 37 | GCAGATCCACGCCATTTAAA | SEQ.ID.NO: 38 |
| 8 | 5 | M3047/1 | M21/22-1 | GCAAACCTTGCATGAACCCGATTGT | SEQ.ID.NO: 21 | CAAGCGTCCAGCTCGATGATTTC | SEQ.ID.NO: 22 |
| 8 | 5 | M3047/1 | M33/34-1 | CAGAGTTGATGAACTGAAAAAGG | SEQ.ID.NO: 33 | CTCTTGCTTCCCCCCTAATC | SEQ.ID.NO: 34 |
| 9 | 7 | M3047/1 | M31/32-1 | GTGAAGAACGATGACGCAGA | SEQ.ID.NO: 31 | CAGCAACGCTCTCACATTGT | SEQ.ID.NO: 32 |
| 9 | 7 | M3047/1 | M39/40-1 | ACAATTCGATCGAGAGCGAG | SEQ.ID.NO: 39 | CCTTTCTTGCTGGTTCTTGC | SEQ.ID.NO: 40 |
| 10 | 7 | M3047/1 | M29/30-1 | TTCCAGTAAGGGAGGTGCTG | SEQ.ID.NO: 29 | TAAGCAACATATAGCCGGGC | SEQ.ID.NO: 30 |
| 11 | 8 | M3047/2 | M67/68-2 | TTGGTGAAACGGTGAAATGA | SEQ.ID.NO: 67 | CTGGTGAGCTTCACCCTCTC | SEQ.ID.NO: 68 |

**Table C QTLs for early and late Root and Stalk Loding, Favorable Alleles and Linked Markers**

| Root/Stalk Lodging QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 1 | M3047/2 | M3/4-2 | TGACGGACGTGGATCGCTTCAC | SEO.ID.NO: 3 | AGCAGGCAGCAGGTCAGCAGCG | SEQ.ID.NO: 4 |
| 1 | 1 | M3047/2 | M59/60-2 | CCCAGCGCATGTCAACTCT | SEO.ID.NO: 59 | CCCCGGTAATTCAGTGGATA | SEQ.ID.NO: 60 |
| 2 | 1 | M3047/2 | M27/28-2 | TTCACCGCCTCACATGAC | SEQ.ID.NO: 27 | GCAACGCTAGCTAGCTTTG | SEQ.ID.NO: 28 |
| 2 | 1 | M3047/2 | M47/48-2 | CAGAAGGGGAGGAGGGATAC | SEQ.ID.NO: 47 | ATTATGCTCAAGCACAGGGC | SEO.ID.NO: 48 |
| 3 | 1 | M3047/1 | M45/46-1 | AGGTCCTGGCACTAAGAGCA | SEO.ID.NO: 45 | AGAGGTGGTATGATCACCTGG | SEQ.ID.NO: 46 |

**Table D QTLs for Common Smut Incidence, Favorable Alleles and Linked Markers**

| Common Smut Incidence QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 3 | M3047/1 | M11/12-1 | TTACTCCTATCCACTGCGGCCTGGAC | SEQ.ID.NO: 11 | GCGGCATCCCGTACAGCTTCAGA | SEO.ID.NO: 12 |

**Table E QTLs for Tassel Architecture, Favorable Alleles and Linked Markers**

| Tassel Architecture QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 3 | M3047/1 | M11/12-1 | TTACTCCTATCCACTGCGGCCTGGAC | SEQ.ID.NO: 11 | GCGGCATCCCGTACAGCTTCAGA | SEQ.ID.NO: 12 |
| 2 | 6 | M3047/1 | M55/56-1 | TTTTCTCCTTGAGTTCGTTCG | SEQ.ID.NO: 55 | ACAGGCAGAGCTCTCACACA | SEQ.ID.NO: 56 |
| 3 | 7 | M3047/1 | M31/32-1 | GTGAAGAACGATGACGCAGA | SEQ.ID.NO: 31 | CAGCAACGCTCTCACATTGT | SEQ.ID.NO: 32 |
| 3 | 7 | M3047/1 | M39/40-1 | ACAATTCGATCGAGAGCGAG | SEQ.ID.NO: 39 | CCTTTCTTGCTGGTTCTTGC | SEQ.ID.NO: 40 |
| 4 | 9 | M3047/1 | M81/82-1 | TGGTCTTCTTCGCCGCATTAT | SEQ.ID.NO: 81 | ATAAGCTCGTTGATCTCCTCCTCC | SEQ.ID.NO: 82 |
| 4 | 9 | M3047/1 | M7/8-1 | GACGTAAGCCTAGCTCTGCCAT | SEQ.ID.NO: 7 | AAACAAGAACGGCGGTGCTGATTC | SEQ.ID.NO: 8 |

**Table F QTLs for Sulcotrione Resistance, Favorable Alleles and Linked Markers**

| Sulcotrione Resistance QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 3 | M3047/2 | M43/44-2 | CTGGGCAGACAGCAACAGTA | SEO.ID.NO: 43 | AGCCAAAGACATGATGGTCC | SEQ.ID.NO: 44 |
| 2 | 9 | M3047/1 | M81/82-1 | TGGTCTTCTTCGCCGCATTAT | SEQ.ID.NO: 81 | ATAAGCTCGTTGATCTCCTCCTCC | SEQ.ID.NO: 82 |
| 2 | 9 | M3047/1 | M7/8-1 | GACGTAAGCCTAGCTCTGCCAT | SEQ.ID.NO: 7 | AAACAAGAACGGCGGTGCTGATTC | SEQ.ID.NO: 8 |

**Table G QTLs for Fusarium Ear Rot Incidence, Favorable Alleles and Linked Markers**

| Fusarium Ear Rot Incidence QTL # | Chromosome # | Favorable Allele | Linked Marker | F1 Primer | F1 Primer Sequence ID Number | R1 Primer | R1 Primer Sequence ID Number |
|---|---|---|---|---|---|---|---|
| 1 | 5 | M3047/1 | M1/2-1 | AGAAAATGGTGAGGCAGG | SEQ.ID.NO: 1 | TATGAAATCTGCATCTAGAAATTG | SEQ.ID.NO: 2 |
| 1 | 5 | M3047/1 | M79/80-1 | AGCTCGAGTACCTGCCGAG | SEQ.ID.NO: 79 | TGCATCTCTGAGACC | SEO.ID.NO: 80 |
| 2 | 5 | M3047/1 | M79/80-1 | AGCTCGAGTACCTGCCGAG | SEQ.ID.NO: 79 | TGCATCTCTGAGACC | SEQ.ID.NO: 80 |
| 2 | 5 | M3047/1 | M15/16-1 | CATGCATCAACGTAACTCCCT | SEQ.ID.NO: 15 | CATGTCACGCGTTCCACTTG | SEQ.ID.NO: 16 |

**Table H: SEQ ID NOs and Nucleotide Sequence of Forward Primers**

| | |
|---|---|
| AGAAAATGGTGAGGCAGG | SEQ.ID.NO: 1 |
| TGACGGACGTGGATCGCTTCAC | SEQ.ID.NO: 3 |
| TAATTCCTCGCTCCCGGATTCAGC | SEQ.ID.NO: 5 |
| GACGTAAGCCTAGCTCTGCCAT | SEQ.ID.NO: 7 |
| CCGAATTGAAATAGCTGCGAGAACCT | SEQ.ID.NO:9 |
| TTACTCCTATCCACTGCGGCCTGGAC | SEQ.ID.NO: 11 |
| CTGCTCTCACTGAGCTTGATGGAAAGG | SEQ.ID.NO: 13 |
| CATGCATCAACGTAACTCCCT | SEQ.ID.NO: 15 |
| CTTCCAGCCGCAACCCTC | SEQ.ID.NO: 17 |
| GGTCACCCTCCCTTTGCAG | SEQ.ID.NO: 19 |
| GCAAACCTTGCATGAACCCGATTGT | SEQ.ID.NO: 21 |
| GATGCAATAAAGGTTGCCGT | SEQ.ID.NO: 23 |
| GGGAGTATGGTAGGGAACCC | SEQ.ID.NO: 25 |
| TTCACCGCCTCACATGAC | SEQ.ID.NO: 27 |
| TTCCAGTAAGGGAGGTGCTG | SEQ.ID.NO: 29 |
| GTGAAGAACGATGACGCAGA | SEQ.ID.NO: 31 |
| CAGAGTTGATGAACTGAAAAAGG | SEQ.ID.NO: 33 |
| CGTTACCCATTCCTGCTACG | SEQ.ID.NO: 35 |
| AGCTGATCTGCACGTTGTTG | SEQ.ID.NO: 37 |
| ACAATTCGATCGAGAGCGAG | SEQ.ID.NO: 39 |
| TTTTCTTTCAAAAATATTCAGAAGC | SEQ.ID.NO: 41 |
| CTGGGCAGACAGCAACAGTA | SEQ.ID.NO: 43 |
| AGGTCCTGGCACTAAGAGCA | SEQ.ID.NO: 45 |
| CAGAAGGGGAGGAGGGATAC | SEQ.ID.NO: 47 |
| AACCAAGGTTCTTGGAGGCT | SEQ.ID.NO: 49 |
| TAATCTTGGGGGGTTTAGGG | SEQ.ID.NO: 51 |
| ACGACTTTCATGCCTCGTCT | SEQ.ID.NO: 53 |
| TTTTCTCCTTGAGTTCGTTCG | SEQ.ID.NO: 55 |
| ACAGCTTTAGACTTAGACCACACG | SEQ.ID.NO: 57 |
| CCCAGCGCATGTCAACTCT | SEQ.ID.NO: 59 |
| TGCTCTCACAAGATGGTGGA | SEQ.ID.NO: 61 |
| ACCGGAACAGACGAGCTCTA | SEQ.ID.NO: 63 |
| ACAGCACTGGGAACCAAAAC | SEQ.ID.NO: 65 |
| TTGGTGAAACGGTGAAATGA | SEQ.ID.NO: 67 |
| TTACGGTACCAATTCGCTCC | SEQ.ID.NO: 69 |
| GAGAAGAGGTGGACAAACTCT | SEQ.ID.NO: 71 |
| TCGTCGTCTCCAATCATACGTG | SEQ.ID.NO: 73 |
| ATATCTTCTTCTTGTCCTCCG | SEQ.ID.NO: 75 |
| TTGCACCCCGTTATTATCCTACAG | SEQ.ID.NO: 77 |
| AGCTCGAGTACCTGCCGAG | SEQ.ID.NO: 79 |
| TGGTCTTCTTCGCCGCATTAT | SEQ.ID.NO: 81 |

**Table I: SEQ ID NOs and Nucleotide Sequence of Reverse Primers**

| | |
|---|---|
| TATGAAATCTGCATCTAGAAATTG | SEQ.ID.NO: 2 |
| AGCAGGCAGCAGGTCAGCAGCG | SEQ.ID.NO: 4 |
| GTGCATGAGGGAGCAGCAGGTAGTG | SEQ.ID.NO: 6 |
| AAACAAGAACGGCGGTGCTGATTC | SEQ.ID.NO: 8 |
| ACAATGAACGGTGGTTATCAACACGC | SEQ.ID.NO: 10 |
| GCGGCATCCCGTACAGCTTCAGA | SEQ.ID.NO: 12 |
| TGCAAATCAATGGCAAGGGACCTCGTAGTT | SEQ.ID.NO: 14 |
| CATGTCACGCGTTCCACTTG | SEQ.ID.NO: 16 |
| CCAACAACGCGGACGTGA | SEQ.ID.NO: 18 |
| ATTGCCTACACAGTTTGATTGG | SEQ.ID.NO: 20 |
| CAAGCGTCCAGCTCGATGATTTC | SEQ.ID.NO: 22 |
| ATGTGCTGTGCCTGCCTC | SEQ.ID.NO: 24 |
| AAACCCTTGGAGCATACCCT | SEQ.ID.NO: 26 |
| GCAACGCTAGCTAGCTTTG | SEQ.ID.NO: 28 |
| TAAGCAACATATAGCCGGGC | SEQ.ID.NO: 30 |
| CAGCAACGCTCTCACATTGT | SEQ.ID.NO: 32 |
| CTCTTGCTTCCCCCCTAATC | SEQ.ID.NO: 34 |
| CTTGCTCGTTTCCATTCCAT | SEQ.ID.NO: 36 |
| GCAGATCCACGCCATTTAAA | SEQ.ID.NO: 38 |
| CCTTTCTTGCTGGTTCTTGC | SEQ.ID.NO: 40 |
| GCAGGATTTCATCGGTTGTT | SEQ.ID.NO: 42 |
| AGCCAAAGACATGATGGTCC | SEQ.ID.NO: 44 |
| AGAGGTGGTATGATCACCTGG | SEQ.ID.NO: 46 |
| ATTATGCTCAAGCACAGGGC | SEQ.ID.NO: 48 |
| ACCATTGTATTTTCCTAGAGAATCG | SEQ.ID.NO: 50 |
| GACATGTCCCATTCCCATTC | SEQ.ID.NO: 52 |
| ATTTCTTTTGCCACCTCAGC | SEQ.ID.NO: 54 |
| ACAGGCAGAGCTCTCACACA | SEQ.ID.NO: 56 |
| GCACAAGCGAAGGTTTTCTC | SEQ.ID.NO: 58 |
| CCCCGGTAATTCAGTGGATA | SEQ.ID.NO: 60 |
| CCACAGGATAAAATCGGCTG | SEQ.ID.NO: 62 |
| GTCCTGCAAAGCAACCTAGC | SEQ.ID.NO: 64 |
| ATCCCCTCTTCCATCTCTGC | SEQ.ID.NO: 66 |
| CTGGTGAGCTTCACCCTCTC | SEQ.ID.NO: 68 |
| GACGACGCCATTTTCTGATT | SEQ.ID.NO: 70 |
| TGGAGGTAGAAGAGAATTGTG | SEQ.ID.NO: 72 |
| GCTACACGATACCATGGCGTTT | SEQ.ID.NO: 74 |
| CATCCCCTTATCCCTCC | SEQ.ID.NO: 76 |
| CCAGACTAGAGTGCCATGATCCTT | SEQ.ID.NO: 78 |
| TGCATCTCTGAGACC | SEQ.ID.NO: 80 |
| ATAAGCTCGTTGATCTCCTCCTCC | SEQ.ID.NO: 82 |

### LIST OF REFERENCES

Edwards et al. (1987) 115 Genetics 113-125
Edwards, M. & Johnson, L. 1994. RFLPs for rapid recurrent selection. Proc. Symp. on Analysis of Molecular Marker Data, pp 33-40. Corvallis, Oregon, American Society of Horticultural Science and Crop Science Society of America.
Fehr, W.R. 1987. Principles of Cultivar Development. Macmillan, New York, New York.
Gallais, A., Dillmann, C. & Hospital, F. 1997. An analytical approach of marker assisted selection with selection on markers only. In R. Krajewski & Z. Kaczmarek, eds. Advances in biometrical genetics. Proc.10th. Meeting of the EUCARPIA Section Biometrics in Plant Breeding, pp 111-116. Poznan, Institute of Plant Genetics, Polish Academy of Sciences.
Gimelfarb, A. & Lande, R. 1994. Simulation of marker-assisted selection in hybrid populations. Genet. Res. 63: 39-47.
Lande, R. & Thompson, R. 1990. Efficiency of marker-assisted selection in the improvement of quantitative traits. Genetics 124: 743-756.
Lander & Schork (1994) 265 Science 2037-2048
Lee, M. 1995. DNA markers in plant breeding programs. Advances in Agronomy, 55: 265-344.
Moreau, L., Charcosset, A., Hospital, F. & Gallais, A. 1998. Marker-assisted selection efficiency in populations of finite size. Genetics 148: 1353-1365.
Peleman, J.D. & Van Der Voort, J.R. 2003. Breeding by design. Trends Plant Sci. 7: 330-334.
Ragot M., Biasiolli, M., Delbut, M F., Dell'Orco, A., Malgarini L., Thevenin, P., Vernoy, J., Vivant, J., Zimmermann, R. & Gay, G. 1995. Marker-assisted backcrossing: a practical example. In A. Bervillé & M. Tersac, eds. Les Colloques, n° 72, Techniques et utilisations des marqueurs moléculaires, pp 45-56. INRA, Paris.
Ribaut, J-M. & Betrán, J. 1999. Single large-scale marker-assisted selection (SLS-MAS). Mol. Breed. 5: 531-541.
Stam, P. 1995. Marker-assisted breeding. In J.W. Van Ooijen & J. Jansen, eds. Biometrics in plant breeding: applications of molecular markers. Proc.9th meeting of the EUCARPIA Section Biometrics in Plant Breeding, pp 32-44. Wageningen, The Netherlands, CPRO-DLO.
Van Berloo, R. & Stam, P. 1998. Marker-assisted selection in autogamous RIL populations: a simulation study. Theor. Appl. Genet. 96: 147-154.
Xie, C. & Xu, S. 1998. Efficiency of multistage marker-assisted selection in the improvement of multiple quantitative traits. Heredity 80: 489-498.
U.S. Patent No 5,385,835
   U.S. Patent No 5,492,547
   U.S. Patent No 5,981,832

## Claims

1. A maize plant containing a nuclear genome comprising a set of alleles at a corresponding set of QTLs each of which contribute to a phenotypic trait of economic importance, wherein
a) each QTL is genetically linked to at least one marker locus, which can be identified by a pair of PCR oligonucleotide primers consisting of a forward primer and a reverse primer exhibiting a nucleotide sequence as given in SEQ ID NO: 1 - 82 shown in Tables A-G; and
b) each allele at the corresponding QTL is defined by at least one marker allele at said at least one marker locus linked to the QTL, which marker allele is **characterized by** the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair; and wherein said
set of QTLs comprises at least 10, particularly at least 15, more particularly at least 20, even more particularly at least 25, but especially at least 30 and up to 37 different QTLs.

2. A maize plant according to claim 1 **characterized by** set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A and as given in SEQ ID NO: 3-6, 9, 10, 13, 14, 21-24, 29-34, 37-40, 43, 44, 53, 54, 57, 58 and 65-72 shown in Table B, wherein said set of QTLs comprises at least 10, particularly at least 15, more particularly at least 20, even more particularly at least 25, different QTLs contributing to the phenotypic trait of grain yield and grain moisture at harvest, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is **characterized by** the PCR amplification product of the respective oligonucleotide primer pair given in Tables A and B, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

3. A maize plant according to claim 2 wherein said set of QTLs comprises 25 different QTLs, 14 of which are contributing to grain yield and mapping to loci on chromosome 1, 2, 4, 5 and 7 and 11 QTLs are contributing to grain moisture mapping to loci on chromosome 1, 2, 3, 4, 5, 7 and 8, wherein each allele at the corresponding QTL is defined by at least one marker allele at said at least one marker locus linked to the QTL, which marker allele is **characterized by** the PCR amplification product of the respective oligonucleotide primer pair given in Tables A and B, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

4. A maize plant according to claim 1 **characterized by** a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B and as given in SEQ ID NO: 3, 4, 27, 28, 45-48, 59 and 60 shown in Table C, wherein said set of QTLs comprises at least 10, particularly at least 15, more particularly at least 20, even more particularly at least 25, but especially at least 28 different QTLs contributing to the phenotypic trait of grain yield, grain moisture at harvest and early and late root lodging, stalk lodging, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is **characterized by** the PCR amplification product of the respective oligonucleotide primer pair given in Tables A-C, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

5. A maize plant according to claim 4 wherein said set of QTLs comprises 28 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, and 3 QTLs are contributing to root and stalk lodging and are mapping to chromosome 1, wherein each allele at the corresponding QTL is defined by at least one marker allele at said at least one marker locus linked to the QTL, which marker allele is **characterized by** the PCR amplification product of the respective oligonucleotide primer pair given in Tables A-C, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

6. A maize plant according to claim 1 **characterized by** a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C and as given in SEQ ID NO: 11 and 12 shown in Table D, wherein the set of QTLs comprises at least 10, particularly at least 15, more particularly at least 20, even more particularly at least 25, but especially at least 29 different QTLs contributing to the phenotypic trait of grain yield, grain moisture at harvest, early and late root lodging, stalk lodging and common smut incidence, which QTLs are mapping to loci on chromosomes 1, 2, 3, 4, 5, 7, and 8, wherein each allele at the corresponding QTL is defined by at least one marker allele at said at least one marker locus linked to the QTL, which marker allele is **characterized by** the PCR amplification product of the respective oligonucleotide primer pair given in Tables A-D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

7. A maize plant according to claim 6 wherein said set of QTLs comprises 29 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, and 1 QTL is contributing to common smut incidence and is mapping to a locus on chromosome 3, wherein each allele at the corresponding QTL is defined by at least one marker allele at said at least one marker locus linked to the QTL, which marker allele is **characterized by** the PCR amplification product of the respective oligonucleotide primer pair given in Tables A-D, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

8. A maize plant according to claim 1 **characterized by** a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, and as given in SEQ ID NO: 7, 8, 11, 12, 31, 32, 39, 40, 55, 56, 81 and 82 shown in Table E, wherein the set of QTLs comprises at least 10, particularly at least 15, more particularly at least 20, even more particularly at least 25, but especially at least 30 and up to 33 different QTLs contributing to the phenotypic trait of grain yield, grain moisture at harvest and early, late root lodging, stalk lodging, common smut incidence and tassel architecture, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is **characterized by** the PCR amplification product of the respective oligonucleotide primer pair given in Tables A-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

9. A maize plant according to claim 8 wherein said set of QTLs comprises 33 different QTLs, 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3 and 4 QTLs are contributing to tassel architecture and are mapping to loci on chromosomes 3, 6, 7 and 9, wherein each allele at the corresponding QTL is defined by at least one marker allele at said at least one marker locus linked to the QTL, which marker allele is **characterized by** the PCR amplification product of the respective oligonucleotide primer pair given in Tables A-E, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair

10. A maize plant according to claim 1 **characterized by** a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, and as given in SEQ ID NO: 7, 8, 43, 44, 81 and 82 shown in Table F, wherein the set of QTLs comprises 35 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, and 2 QTLs are contributing to sulcotrione resistance and are mapping to loci on chromosomes 3 and 9, wherein each allele at the corresponding QTL is defined by at least one marker allele at said at least one marker locus linked to the QTL, which marker allele is **characterized by** the PCR amplification product of the respective oligonucleotide primer pair as shown pintables A-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

11. A maize plant according to claim 10 wherein said set of QTLs comprises at least 10, particularly at least 15, more particularly at least 20, but especially at least 21 different QTLs contributing to the phenotypic trait of grain moisture at harvest and early, late root lodging, stalk lodging, common smut incidence, tassel architecture and sulcotrione resistance, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is **characterized by** the PCR amplification product of the respective oligonucleotide primer pair given in Tables B-F, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

12. A maize plant according to claim 1 **characterized by** a set of alleles at a corresponding set of QTLs, with each QTL being genetically-linked to at least one marker locus, which can be identified by a pair of PCR oligonucleotide primers consisting of a forward primer and a reverse primer exhibiting a nucleotide sequence as given in SEQ ID NO: 9, 10, 13, 14, 17-20, 25-30, 35, 36, 41, 42, 47-52, 59-66, 69, 70 and 73-78 shown in Table A; as given in SEQ ID NO: 3-6, 9, 10, 13, 14, 21-24, 29-34, 37-40, 43, 44, 53, 54, 57, 58 and 65-72 shown in Table B, as given in SEQ ID NO: 3, 4, 27, 28, 45-48, 59 and 60 shown in Table C, as given in SEQ ID NO: 11 and 12 shown in Table D, as given in SEQ ID NO: 7, 8, 11, 12, 31, 32, 39, 40, 55, 56, 81 and 82 shown in Table E, as given in SEQ ID NO: 7, 8, 43, 44, 81 and 82 shown in Table F, and as given in SEQ ID NO: 1, 2, 15, 16, 79 and 80 shown in Table G, wherein the set of QTLs comprises 37 different QTLs , 14 of which are contributing to grain yield and are mapping to loci on chromosome 1, 2, 4, 5 and 7; 11 QTLs are contributing to grain moisture and are mapping to loci on chromosome 1, 2, 3, 4, 5, 7 and 8, 3 QTLs are contributing to root and stalk lodging and are mapping to chromosome 1, 1 QTL is contributing to common smut incidence and is mapping to a locus on chromosome 3, 4 QTLs are contributing to tassel architecture and are mapping to loci on chromosomes 3, 6, 7 and 9, 2 QTLs are contributing to sulcotrione resistance and are mapping to loci on chromosomes 3 and 9, and 2 QTLs are contributing to fusarium ear rot incidence and are mapping to loci on chromosome 5, wherein each allele at the corresponding QTL is defined by at least one marker allele at said at least one marker locus linked to the QTL, which marker allele is **characterized by** the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

13. A maize plant according to claim 12 wherein said set of QTLs comprises at least 10, particularly at least 15, more particularly at least 20, but especially at least 23 different QTLs contributing to the phenotypic trait of grain moisture at harvest and early, late root lodging, stalk lodging, common smut incidence, tassel architecture, sulcotrione resistance and fusarium ear rot incidence, which QTLs are mapping to loci on chromosomes 1, 2, 3, 4, 5, 6, 7, 8 and 9, wherein each allele at the corresponding QTL is defined by at least one marker allele at said at least one marker locus linked to the QTL, which marker allele is **characterized by** the PCR amplification product of the respective oligonucleotide primer pair given in Tables B-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair.

14. A maize plant containing a nuclear genome comprising a defined set of alleles at a defined set of QTLs each of which contributes to a phenotypic trait selected from the group of grain yield, grain moisture at harvest, early and late root lodging, stalk lodging, common smut incidence, fusarium ear rot incidence, sulcotrione resistance, and tassel architecture, wherein
a) each QTL is genetically linked to at least one marker locus, which can be identified by a pair of PCR oligonucleotide primers consisting of a forward primer and a reverse primer exhibiting a nucleotide sequence as given in SEQ ID NO: 1 - 82 shown in Tables A-G; and
b) each allele at the corresponding QTL is defined by at least one marker allele at said at least one marker locus linked to the QTL, which marker allele is **characterized by** the PCR amplification product of the respective oligonucleotide primer pair given in Tables A-G, which amplification product is essentially identical to the corresponding amplification product of the favourable allele as indicated in Tables A-G obtainable from inbred lines M3047/1 (NCIMB 41459) and M3047/2 (NCIMB 41460) in a PCR reaction with the identical primer pair; and wherein said
set of QTLs comprises 37 different QTLs as given in Tables A-G.

15. A maize plant according to any of the preceding claims which plant is
i. an inbred
ii. a hybrid.

16. A maize plant according to any of the preceding claims, which plant always carries the most favourable allele at the marker loci linked to the QTL and/or exhibits a LOT score as given in Tables A-G.

17. A maize plant according to any one of the preceding claims wherein at least part of the recited QTLs are obtained from maize inbred lines M3047/2 and M3047/1, respectively, deposited with NCIMB under accession number NCIMB 41460 and NCIMB 41459.

18. Plant material including plant parts and plant seed and processed maize products, particularly maize grains and kernels obtainable from a plant according to any of the preceding claims.

19. Processed maize products, particularly maize grains and kernels obtainable from a plant according to any of the preceding claims.

20. A method of producing a plant according to any of the preceding claims comprising the steps of
a. crossing two or more parent plants at least one of which has a genetic background capable of contributing to the development of a plant according to the invention and as described herein before, particularly crossing two parent plants which have a genetic background as represented by maize inbred lines M3047/1 (NCIMB 41459) and M3047/2 (NCIMB 41460), or an ancestor or progenitor plant thereof, and optionally identifying superior performing by phenotypic selection from the progeny plant population resulting from the cross performed in step a.) and selfing said plants to produce a 2^{nd} breeding population;
b. optionally carrying out further selfing and selection cycles according to step a)
c. screening for a plant which has in its genome a set of alleles at a corresponding set of QTLs, with each QTL being genetically-linked to at least one marker locus, wherein said set of QTLs comprises at least 10, particularly at least 15, more particularly at least 20, even more particularly at least 25, but especially at least 30 and up to 37 different QTLs, wherein each allele at the corresponding QTL is defined by at least one marker allele at said at least one marker locus linked to the QTL by
d. identifying the at least one marker locus in a PCR reaction using a pair of PCR oligonucleotide primers consisting of a forward primer and a reverse primer exhibiting a nucleotide sequence as given in SEQ ID NO: 1-82 shown in Table A-G, and
e. identifying the marker allele by determining the molecular weight of the PCR amplification product obtained in step 1.
f. selecting a plant with the desired profile.

21. A set of two or more markers and up to 41 markers comprising a pair of PCR oligonucleotide primers consisting of a forward primer and a reverse primer exhibiting a nucleotide sequence as given in SEQ ID NO: 1-82 shown in Tables A-G, which primers lead to an amplification product in a PCR reaction exhibiting a molecular weight or a nucleotide sequence, which is essentially identical to that of a corresponding PCR amplification product obtainable from inbred lines M3047/1 (NCIMB 41459) and M3047/2 (NCIMB 41460) in a PCR reaction with the identical primer pair.

22. A method of using a set of nucleic acid markers according to claim 21 in marker-based selection for introgressing a set of alleles which are associated to a corresponding set of QTLs into maize germplasm lacking said set of alleles, wherein said alleles contribute to a phenotypic trait selected from the group of grain yield, grain moisture at harvest, early and late root lodging, stalk lodging, common smut incidence, fusarium ear rot incidence, sulcotrione resistance, and tassel architecture and the nucleic acid markers are selected from the group of markers shown in Tables A-G.

23. A method of identifying a maize plant according to any of the preceding claims, which method comprises the following steps:
i) obtaining plant material from a plant or a plant population to be tested and extracting DNA from said material;
ii) analyzing the DNA sample obtained in step i) to determine the allelic variants present at at least 1, particularly at al least 5, more particularly at at least 15, even more particularly at at least 20, but especially at at least 25 and up to 30-40 marker loci genetically linked to a corresponding QTL contributing to a phenotypic trait selected from the group of grain yield, grain moisture at harvest, early and late root lodging, stalk lodging, common smut incidence, fusarium ear rot incidence, sulcotrione resistance, and tassel architecture, by
a) identifying the at least one marker locus in a PCR reaction using a pair of PCR oligonucleotide primers consisting of a forward primer and a reverse primer exhibiting a nucleotide sequence as given in SEQ ID NO: 1-82 shown in Table A-G, and
b) identifying the marker allele by determining the molecular weight of the PCR amplification product obtained in step 1.
iii) identifying a plant or plants with the desired profile using the data of the marker analysis.
